(19) Europäisches Patentamt

European Patent Office

Office européen des brevets

(11) **EP 1 589 092 A1**

(12) **EUROPEAN PATENT APPLICATION**

(43) Date of publication:
**26.10.2005 Bulletin 2005/43**

(51) Int Cl.[7]: **C11D 3/00**, B01J 13/00, A61K 7/50, C11D 3/50, A61K 7/16, A61K 7/46

(21) Application number: **05252298.4**

(22) Date of filing: **13.04.2005**

| | |
|---|---|
| (84) Designated Contracting States: **AT BE BG CH CY CZ DE DK EE ES FI FR GB GR HU IE IS IT LI LT LU MC NL PL PT RO SE SI SK TR** Designated Extension States: **AL BA HR LV MK YU** | (72) Inventors: • **Lee, Kaiping Morganville, New Jersey 07751 (US)** • **Popplewell, Lewis Michael Morganville, New Jersey 07751 (US)** |
| (30) Priority: **13.04.2004 US 823033** | (74) Representative: **Mercer, Christopher Paul et al Carpmaels & Ransford, 43-45 Bloomsbury Square London WC1A 2RA (GB)** |
| (71) Applicant: **INTERNATIONAL FLAVORS & FRAGRANCES INC. New York New York 10019 (US)** | |

(54) **Stable Fragrance microcapsule suspension and process for using same**

(57)     Described is a stable initial impact and continuous impact fragrance and/or benefit agent- imparting aqueous suspension of microencapsulated fragrance and/or benefit agent, e.g., malodour counteractant suspended in a non-confined fragrance-containing and/or benefit agent-containing liquid phase oil-in-water emulsion. On storage, the viscosity of the suspension undergoes a minimal increase over an extended period of time thereby avoiding undesirable agitation resistance during the blending of the suspension with other materials.

The suspension is thus useful for imparting a benefit or an aroma to a consumable material such as a liquid anionic, cationic, non-ionic or zwitterionic detergent, a shampoo, a bodywash, liquid soaps, hair conditioners, skin lotions, antiperspirants, deodorants or liquid fabric softener and/or conditioner compositions. Also described is a process for preparing such stable suspensions and apparatus for carrying out such process.

**Description**

## FIELD OF THE INVENTION

Stable substantially constant viscosity suspensions of fragrance and/or benefit agent-containing microcapsules, suspended in a non-confined fragrance and/or benefit agent-emulsifier-water oil-in-water emulsion.

## BACKGROUND OF THE INVENTION

The need for controlled and, in many cases, targeted delivery of fragrances and benefit agents, e.g., malodour counteractants to fabrics, the human epidermis, to hair follicle groups and to the environment proximate thereto with at least a modicum of permanency together with the need for simultaneously providing an initial burst of fragrance and/or benefit agent is well-recognized in the prior art. In addition, the need for viscosity control of household aqueous products, e.g., liquid fabric softener and/or fabric conditioner compositions, during preparation and during use thereof is well-recognized in the prior art.

Application to skin of colognes, after-shave lotions, after-bath preparations and splash lotions containing fragrance-containing microcapsules (including those having walls fabricated from urea-formaldehyde polymers) and non-confined fragrances in conjunction with a suspending agent such as a clay is disclosed in U.S. Patent 4,428,869. Utilization of a suspension of fragrance-containing microcapsules (including those having walls fabricated from aminoplast polymers) and non-confined fragrances in conjunction with a suspension agent such as clay for inclusion in liquid or solid fabric softener compositions is disclosed in U.S. Patents 4,464,271 and 4,446,032. Stable fabric softening compositions containing water and a non-confined fragrance oil and an encapsulated fragrance or benefit agent which are combined and then added to a cationic softening compound are disclosed in U.S. Patent 6,620,777. Perfume compositions wherein different scent notes are successively released, initially from a cosmetic gel and then from a microcapsule are disclosed in U.S. Patent 6,653,277. Moisturizing creams containing non-confined fragrance and microencapsulated fragrance (including those having walls fabricated from aminoplast polymers) which are combined and added to an emulsion and gels containing non-confined fragrance and microencapsulated fragrance (including those having walls fabricated from aminoplast polymers) are disclosed in Application for U.S. Patent Serial Number 10/776,298 filed on February 11, 2004. In addition, ingestible flavored substances containing non-confined flavor essences to yield a flavor burst effect and microencapsulated controlled-release flavor essences are disclosed in published European Patent Application 0 437 098 A2 published on July 17, 1991 and abstracted in Chem. Abstracts, 116: 127348q.

Furthermore, microencapsulated fragrances and other benefit agents have been used in consumer products to improve fragrance deposition, retention and longevity. However in order to provide other fragrance benefits to the product or in use, it has been found to be desirable to employ a non-confined fragrance along with employment of the encapsulated fragrance. Thus for example, where a product being consumer-marketed at a point-of-purchase contains a fragrance that is contained in a plurality of microcapsules the walls of which have a low magnitude of porosity, the microencapsulated fragrance will provide little contribution to the headspace at the point of purchase. If the point-of-purchase aroma is important to the consumer purchase decision, then a need exists to add non-confined fragrance to the product in order to optimize both the aroma intensity and the hedonics.

In each of the aforementioned situations, two separate fragrance components are used as follows: (a) a microencapsulated fragrance and (b) a non-confined fragrance, each of which is attempted to be introduced into the commercial product, frequently in combination with polymeric deposition aids. Use of the prior art compositions and techniques as set forth supra has resulted in the problem of product destabilization wherein the microencapsulated fragrance, and the water which is present separates from the non-confined fragrance, even in the presence of a suspension agent.

Techniques for avoiding or overcoming such problems are neither expressly nor implicitly disclosed in the prior art.

Attempts to control viscosity of aqueous household products are set forth in the prior art, for example, in U. S. Patent 6,667,287 which relates to a toilet bowl light duty liquid cleaning composition containing at least one surfactant, a disinfecting agent, a fragrance composition, water and a polymeric viscosity modifier such as a quaternary ammonium polyacrylic acid homopolymer.

## SUMMARY OF THE INVENTION

Our invention is directed to a stable suspension of microencapsulated fragrance and/or benefit agent such as malododour counteractant or insect repellent in an aqueous emulsion of non-confined fragrance and/or benefit agent. The suspensions of our invention are useful as a fragrance modifier or additive or as a benefit agent, e.g.,

malodour counteractant or insect repellent, additive to various consumable articles including but not limited to liquid anionic, cationic, non-ionic or zwitterionic detergents, shampoos, bodywashes, soaps, hair conditioners, skin lotions, skin creams, skin moisturizers, anti-perspirants, deodorants and liquid fabric softener and/or fabric conditioner compositions.

The aqueous emulsion in which the microcapsules are suspended in addition to containing water and fragrance and/or benefit agent, also contains an emulsifier having a HLB ("hydrophile-lipophile balance") of from about 6 to about 40, with the provisos that:

(a) when using a non-ionic emulsifier the HLB value is in the range of from about 6 to about 20;
(b) when using an anionic emulsifier, the HLB value is in the range of from about 10 to about 40; and
(c) when using a zwitterionic emulsifier, the HLB value is in the range of from about 6 to about 12.

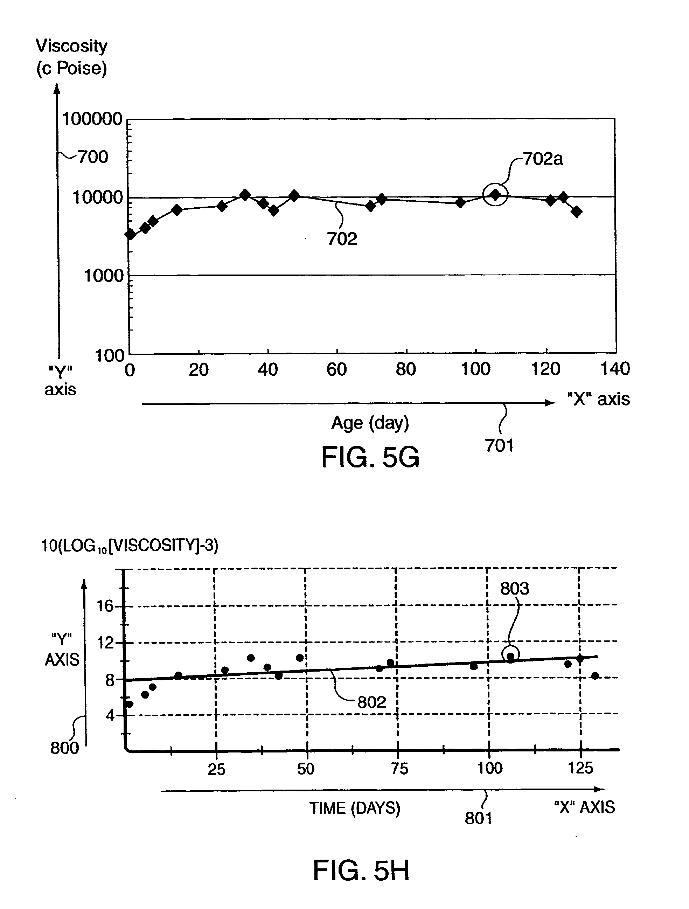

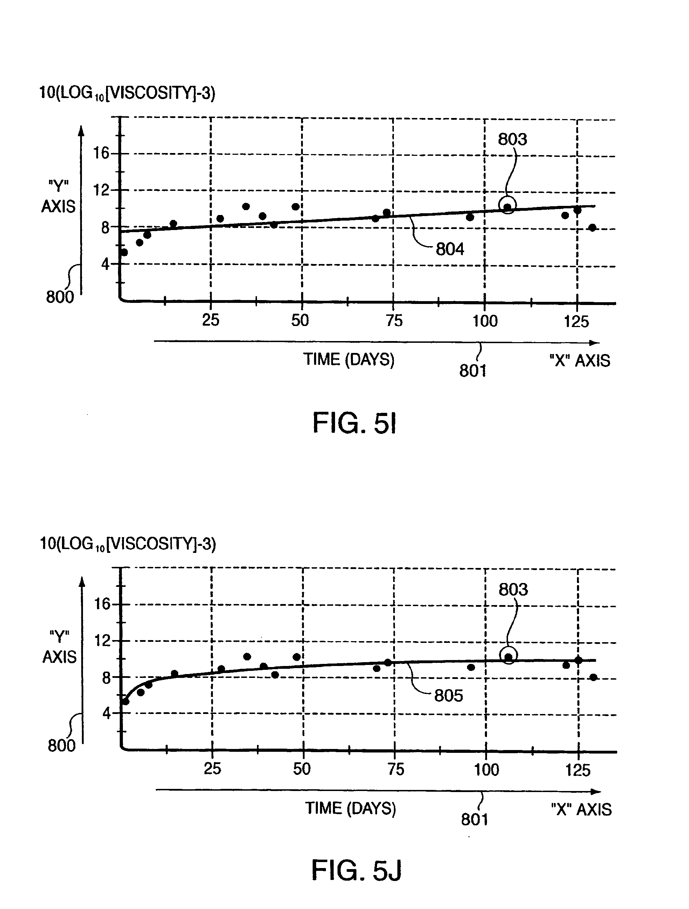

The stable suspension of our invention has a viscosity of from about 10000 to about 20,000 centipoises at a shear rate of from about 0.5 to about 2.0 seconds$^{-1}$ and at about 25°C which viscosity undergoes a minimal increase over an extended period of time on storage, prior to being admixed with the consumable article with which it is to be used, as shown in Figures 5G, 5H, 5I and 5J. Such minimal increase in viscosity of the suspension of our invention on storage over an extended period of time is preferably in accordance with the set of algorithms:

(i) $\log_e v = \alpha\theta + \beta$ and $\frac{\partial v}{\partial \theta} = \alpha v$;

(ii) $\log_e v = \gamma e^{\delta\theta} + \varepsilon$ and $\frac{\partial v}{\partial \theta} = v\delta\gamma e^{\delta\theta}$; and

(iii)

$$\log_e v = \kappa \log_e \theta + \lambda \text{ and } \frac{\partial v}{\partial \theta} = \kappa\left(\frac{v}{\theta}\right)$$

wherein:

$0.003 \leq \alpha \leq 0.006$;
$7 \leq \beta \leq 10$;
$1 \leq \gamma \leq 3$;
$0.002 \leq \delta \leq 0.003$;
$6 \leq \varepsilon \leq 8$;
$0.15 \leq \kappa \leq 0.25$; and
$7 \leq \lambda \leq 9$

and wherein $v$ represents the viscosity of said suspension in units of centipoises and $\theta$ represents the time of storage of said suspension immediately subsequent to production of said suspension, in terms of days.

The term "stable suspension" is herein intended to mean a suspension of microencapsulated fragrance and/or benefit agent in an aqueous oil-in-water emulsion of non-confined fragrance and/or benefit agent where, on storage, over an extended period of time, no settling or precipitation of the microencapsulated fragrance and/or benefit agent occurs and the emulsion surrounding the microcapsules remains as a stable emulsion in the absence of separation into finite discrete non-emulsified liquid phases, an aqueous phase and an oil phase.

More specifically, the suspension of our invention comprises (a) from about 10% by weight to about 90% by weight of a non-confined liquid-phase which is a substantially solid particle-free first fragrance composition and/or a substantially solid particle-free first benefit agent composition comprising from about 10% to about 90% by weight of a fragrance and/or benefit agent, from about 0.5% to about 10% of an emulsifier based on the weight of the non-confined fragrance and from about 10% to about 90% water, in the form of a stable oil-in-water emulsion and (b) stably suspended in said non-confined liquid-phase from about 10% to about 90% by weight of a plurality of microcapsules, the microcapsules in a preferred embodiment are friable, particularly when the microcapsules are dry, the microcapsules of which (i) has an outside diameter in the range of from about 0.01 to about 1000 microns; (ii) has a wall thickness in the range of from about 0.01 to about 100 microns; (iii) has a wall composed of a polymer; and (iv) has a liquid phase core, containing preferably a monophasic core comprising a substantially solid particle-free second fragrance composition and/or substantially solid particle-free second benefit agent composition with the composition of each of the monophasic cores of each of said microcapsules being (A) the same and/or different from one another and (B) the same or different from the first fragrance composition and/or first benefit agent composition wherein the weight % of substantially solid particle-free second fragrance composition and/or substantially solid particle-free second benefit agent composition initially contained in each of the microcapsules is from about 5% to 90% by weight of the microcapsules.

Our invention is also directed to a process for preparing such stable suspensions comprising the steps of (A) providing an aqueous slurry of a plurality of microcapsules having a polymeric wall and a core comprising a first fragrance composition and/or at least one first benefit agent; (B) admixing a non-ionic, anionic or zwitterionic emulsifier having a HLB value of from about 6 to about 40, with the provisos that:

(a) when using a non-ionic emulsifier the HLB value is in the range of from about 6 to about 20;
(b) when using an anionic emulsifier, the HLB value is in the range of from about 10 to about 40; and
(c) when using a zwitterionic emulsifier, the HLB value is in the range of from about 6 to about 12

with a second hydrophobic fragrance composition and/or a second hydrophobic benefit agent thereby forming an emulsifier-second fragrance and/or second benefit agent mixture; and (C) admixing the aqueous slurry with the emulsifier-second fragrance and/or second benefit agent mixture.

Our invention is also directed to apparatus useful for carrying out the process of our invention comprising:

(i) slurry preparation means for preparing a slurry of microencapsulated fragrance and/or benefit agent in water comprising (a) homogenization means, (b)fragrance and/or benefit agent-hydrophobic solvent first mixing means which is associated with and upstream from said homogenization means; (c) polymer-cross-linking agent reaction means which is associated with and upstream from said homogenization means, and (d) microcapsule wall curing means for forming cured microencapsulated fragrance and/or benefit agent downstream from and associated with said homogenization means;
(ii) high shear second mixing means downstream from and associated with said curing means in which said stable suspension is formed;
(iii) means for introduction of the cured microencapsulated fragrance and/or benefit agent from the curing means into the high shear second mixing means;
(iv) third mixing means separate from the slurry preparation means for mixing emulsifier and non-confined fragrance and/or benefit agent, whereby a second mixture is formed;
(v) means for second mixture introduction into the high shear second mixing means and
(vi) optional storage means for storing the stable suspension formed in said high shear second mixing means, with the optional storage means being located downstream from and associated with the high shear second mixing means.

Our invention is also directed to the use of the aforementioned suspensions of our invention as a fragrance modifier or additive or as a benefit agent, e.g., malodour counteractant or insect repellent, additive to various consumable articles including but not limited to liquid anionic, cationic, non-ionic or zwitterionic detergents, shampoos, bodywashes, soaps, hair conditioners, skin lotions, skin creams, skin moisturizers, anti-perspirants, deodorants and liquid fabric softener and/or fabric conditioner compositions. Thus, our invention encompasses compositions comprising such consumable articles and processes for preparing such compositions.

## DETAILED DESCRIPTION OF THE INVENTION

The stable suspension of our invention comprises a suspension of (i) a plurality of microcapsules filled with a fragrance and/or other benefit agent, e.g., malodour counteractant and/or insect repellent optionally in admixture with a compatible solvent in (ii) an aqueous emulsion comprising a non-confined fragrance, water and an aqueous emulsion comprising an emulsifier having a HLB value of from about 6 to about 40 with the provisos that:

(a) when using a non-ionic emulsifier the HLB value is in the range of from about 6 to about 20;
(b) when using an anionic emulsifier, the HLB value is in the range of from about 10 to about 40; and
(c) when using a zwitterionic emulsifier, the HLB value is in the range of from about 6 to about 12.

## The Emulsifier

For the purpose of creation of the suspensions of our invention the emulsifiers, also termed 'surfactants' are employed in a concentration of from about 0.5% to about 100% by weight based on the amount of non-confined fragrance composition and/or benefit agent; preferably from about 1% to about 10% by weight based on the amount of non-confined fragrance and/or benefit agent, and most preferably at about 2.5% by weight based on the amount of non-confined fragrance composition and/or benefit agent. As indicated, among the emulsifiers that may be employed are (a) non-ionic emulsifiers having HLB values in the range of from about 6 to about 20, a number of examples of which are set forth in the following Table Ia together with their respective HLB values:

Table Ia

| Common Name("TWEEN" , "SPAN" and "ATLAS" are the registered trademarks of ICI Americas Inc. of Bridgewater, N.J.) | Chemical Designation | HLB Value |
|---|---|---|
| SPAN 40 | Sorbitan monpalmitate | 6.7 |
| ATLAS G-2800 | Polyoxypropylene mannitol dioleate | 8.0 |
| PEG 400 monolaurate | polyoxyethylene monolaurate | 13.1 |
| TWEEN 60 | polyoxyethylene sorbitan monostearate | 14.9 |
| TWEEN 40 | polyoxyethylene sorbitan monopalmitate | 15.6 |
| TWEEN 20 | polyoxyethylene sorbitan monolaurate | 16.7 |
| ATLAS G-2159 | polyoxyethylene monostearate | 18.8 |

(b) anionic emulsifiers having HLB values in the range of from about 10 to about 40, a number of examples of which are set forth in the following Table Ib together with their respective HLB values:

Table Ib

| Common Name | Chemical Name | HLB Value |
|---|---|---|
| ATLAS G-3300 | An alkyl aryl sulfonate | 11.7 |
| Triethanolamine oleate | Triethanolamine oleate | 12 |
| Sodium Oleate | Sodium Oleate | 18 |
| Potassium Oleate | Potassium Oleate | 20 |
| Sodium Lauryl Sulfate | Sodium Lauryl Sulfate | 40 |

(c) zwitterionic emulsifiers having HLB values in the range of from about 6 to about 12, which are lecithins containing one or more phosphatidyl cholines, phosphatadylethanolamines and/or phosphatidylinositols, a number of examples of which are set forth in the following Table Ic, together with their respective HLB values:

Table Ic

| Common Name (All Registered Trademarks of Central Soya Company Inc. of Fort Wayne, Indiana) | HLB Value |
|---|---|
| Centrophase HR 4B | 7.5 |
| Blendmax K | 8.0 |
| Centrolene A | 10 |
| Centromix E | 12 |
| Centromix CPS | 12 |

(d) polymeric emulsifiers, a number of examples of which are set forth in the following Table Id:

## Table Id

| Name |
|---|
| Gum Arabic, Colloides Naturale |
| Capsul Modified Starch, National Starch & Chemical |
| Pemulen, Noveon Inc., a high molecular weight, cross linked copolymers of acrylic acid and a hydrophobic comonomer |

**The Viscosity of the Stable Suspension**

The viscosity of the stable suspension of our invention is in the range of from about 100 to about 20,000 centipoises at a shear rate of from about 0.5 to about 2.0 seconds[-1] and at about 25°C; preferably from about 1,000 to about 15,000 centipoises at a shear rate of from about 0.5 to about 2.0 seconds[-1] and at about 25°C; and more preferably from about 2,000 to about 12,000 centipoises at a shear rate of from about 0.5 to about 2.0 seconds[-1] and at about 25°C. When compared with a system that has no emulsifier having a HLB value of from about 6 to about 40, with the aforementioned provisos, the viscosity of the suspension of our invention undergoes a minimal increase over an extended period of time during storage, prior to admixing of the suspension with a consumable material base with which it is to be used.

With reference to the viscosity of the suspension of our invention undergoing a "minimal increase over an extended period of time", the term "minimal increase over an extended period of time" is herein intended to mean: "a maximum rate of viscosity increase of about 25 centipoises (at a shear rate of from about 0.5 to about 2.0 seconds[-1] and at about 25°C) per day for a period of time of greater than about 10 days".

Referring to the set of algorithms: $\log_e v = \alpha\theta + \beta$ and $\frac{\partial v}{\partial \theta} = \alpha v$, the range of ratios of the rate of change of the viscosity of the suspension not containing emulsifier having a HLB in the range of from about 6 to about 40, with the aforementioned provisos, with respect to time: the rate of change of the viscosity of the suspension containing emulsifier having a HLB in the range of from about 6 to about 40, with the aforementioned provisos, with respect to time (hereinafter indicated as:

$$\left(\frac{\partial v}{\partial \theta}\right)_{w/oEM} : \left(\frac{\partial v}{\partial \theta}\right)_{withEM} )$$

is from about 8:1 to about 27:1.

Referring to the set of algorithms: $\log_e v = \gamma e^{\delta\theta} + \varepsilon$ and $\frac{\partial v}{\partial \theta} = v\delta\gamma e^{\delta\theta}$, the range of ratios of the range of change of the viscosity of the suspension not containing emulsifier having a HLB in the range of from about 6 to about 40, with the aforementioned provisos, with respect to time: the rate of change of the viscosity of the suspension containing an emulsifier having a HLB in the range of from about 6 to about 40, with the aforementioned provisos, with respect to time (hereinafter indicated as:

$$\left(\frac{\partial v}{\partial \theta}\right)_{w/oEM} : \left(\frac{\partial v}{\partial \theta}\right)_{withEM}$$

is from about 15:1 to about 45:1.

Referring to the set of algorithms

$$\log_e v = \kappa \log_e \theta + \lambda \text{ and } \frac{\partial v}{\partial \theta} = \kappa \left( \frac{v}{\theta} \right),$$

the range of ratios of the range of change of the viscosity of the suspension not containing emulsifier having a HLB in the range of from about 6 to about 40, with the aforementioned provisos, with respect to time: the rate of change of the viscosity of the suspension containing an emulsifier having a HLB in the range of from about 6 to about 40, with the aforementioned provisos, with respect to time (hereinafter indicated as:

$$\left( \frac{\partial v}{\partial \theta} \right)_{w/oEM} : \quad \left( \frac{\partial v}{\partial \theta} \right)_{withEM} )$$

is from about 3.5:1 to about 40:1.

**The Microcapsules**

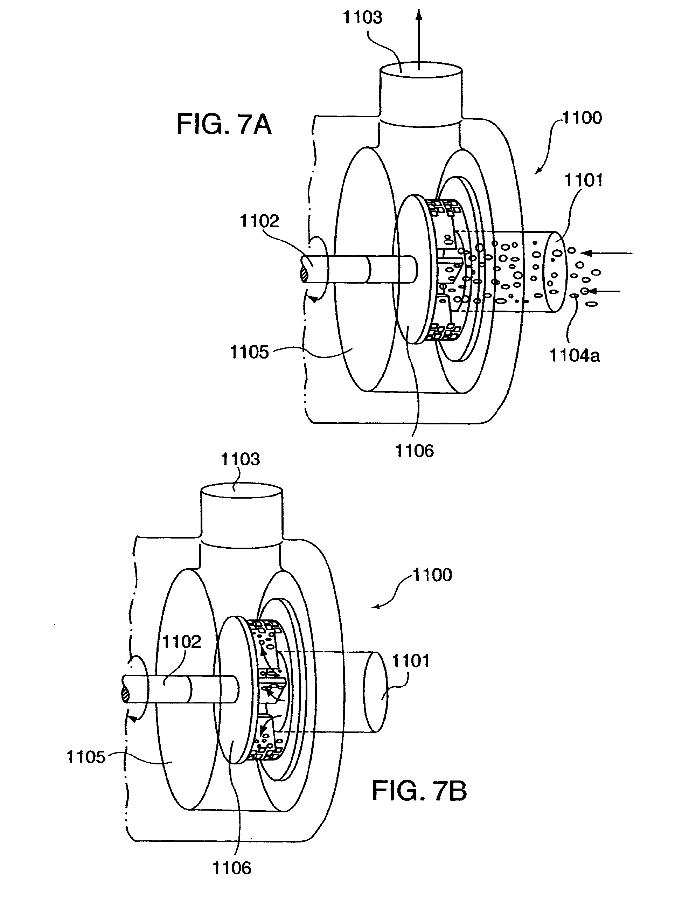

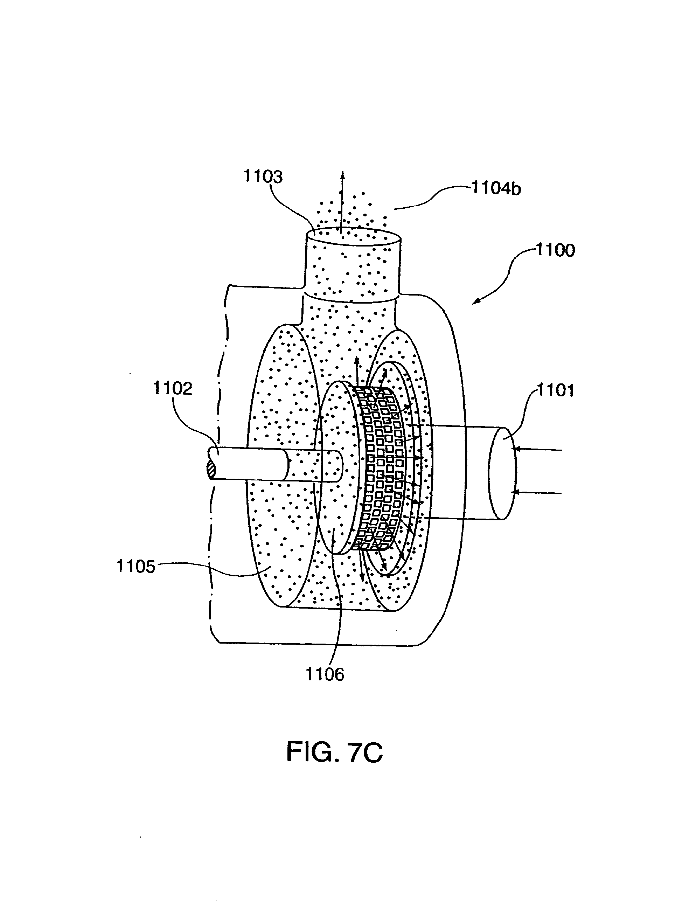

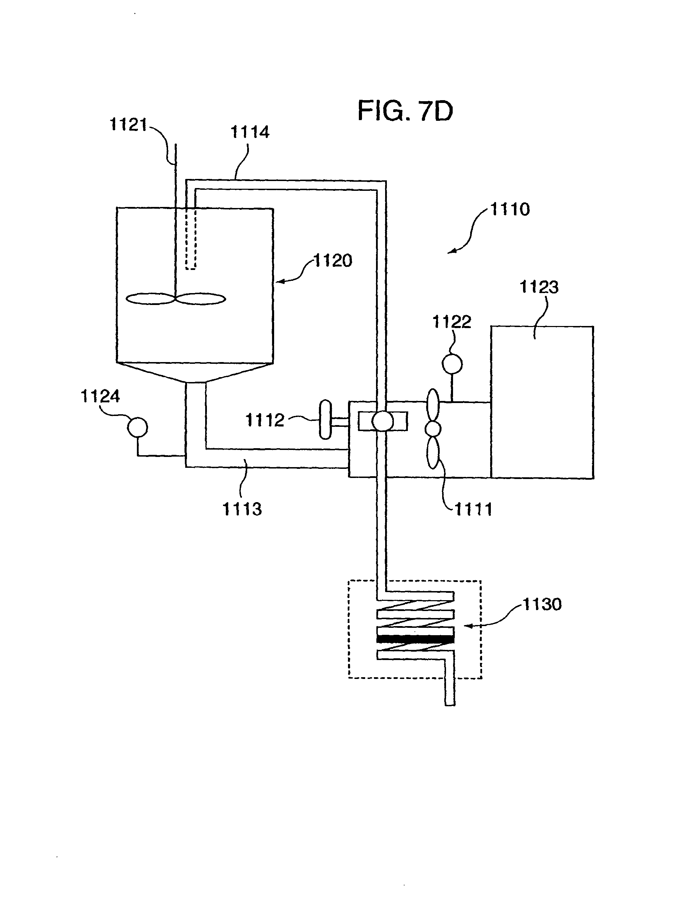

The microcapsule walls are preferably composed of an aminoplast resin, more specifically a substituted or un-substituted acrylic acid polymer or co-polymer cross-linked with a urea-formaldehyde pre-condensate or a melamine-formaldehyde pre-condensate. The microcapsule is formed by means of either (a) forming an aqueous dispersion of a non-cured aminoplast resin by reacting under acidic pH conditions a urea-formaldehyde pre-condensate or a melamine-formaldehyde pre-condensate with one or more substituted or un-substituted acrylic acid polymers or co-polymers; then coacervating the resulting non-cured aminoplast resin shell about the surface of a fragrance and/or malodour counteractant-solvent monophasic droplet under homogenization conditions, e.g., using a homogenization apparatus as described in U.S. Patent 6,042,792 and illustrated in Figures 7A, 7B, 7C and 7D, and then curing the microcapsule shell wall at an elevated temperature, e.g., 50 - 85°C or (b) forming the aminoplast resin wall at the surface of the fragrance and/or malodour counteractant - solvent monophasic droplet by means of reacting, at the surface of the droplet a urea-formaldehyde pre-condensate or a melamine-formaldehyde pre-condensate with one or more substituted or un-substituted acrylic acid polymers or co-polymers, and then curing the microcapsule shell wall at an elevated temperature, e.g., 50 - 85°C.

Microcapsule formation using mechanisms similar to the foregoing mechanism, using (i) melamine-formaldehyde or urea-formaldehyde pre-condensates and (ii) polymers containing substituted vinyl monomeric units having proton-donating functional group moieties, e.g., sulfonic acid groups or carboxylic acid anhydride groups, bonded thereto is disclosed in U.S. Patent 4,406,816 (2-acrylamido-2-methyl-propane sulfonic acid groups), UK published Patent Application GB 2,062,570 A (styrene sulfonic acid groups) and UK published Patent Application GB 2,006,709 A (carboxylic acid anhydride groups).

When substituted or un-substituted acrylic acid co-polymers are employed in the practice of our invention, in the case of using a co-polymer having two different monomeric units, e.g., acrylamide monomeric units and acrylic acid monomeric units, the mole ratio of the first monomeric unit to the second monomeric unit is in the range of from about 1:9 to about 9:1, preferably from about 3:7 to about 7:3. In the case of using a co-polymer having three different monomeric units, e.g., ethyl methacrylate, acrylic acid and acrylamide, the mole ratio of the first monomeric unit to the second monomeric unit to the third monomeric unit is in the range of 1: 1: 8 to about 8:8: 1, preferably from about 3: 3:7 to about 7:7:3.

The molecular weight range of the substituted or un-substituted acrylic acid polymers or co-polymers useful in the practice of our invention is from about 5,000 to about 1,000,000, preferably from about 10,000 to about 100,000. The substituted or un-substituted acrylic acid polymers or co-polymers useful in the practice of our invention may be branched, linear, star-shaped, dendritic-shaped or may be a block polymer or copolymer, or blends of any of the aforementioned polymers or copolymers.

Such substituted or un-substituted acrylic acid polymers or co-polymers may be prepared according to any processes known to those skilled in the art, for example, U.S. Patent 6,545,084.

The urea-formaldehyde and melamine-formaldehyde pre-condensate microcapsule shell wall precursors are prepared by means of reacting urea or melamine with formaldehyde where the mole ratio of melamine or urea to formaldehyde is in the range of from about 10:1 to about 1:6, preferably from about 1:2 to about 1:5. For purposes of practicing our invention, the resulting material has a molecular weight in the range of from 156 to 3000. The resulting material may be used 'as a cross-linking agent for the aforementioned substituted or un-substituted acrylic acid polymer or copolymer or it may be further reacted with a $C_1$-$C_6$ alkanol, e.g., methanol, ethanol, 2-propanol, 3-propanol, 1-butanol , 1-pentanol or 1-hexanol, thereby forming a partial ether where the mole ratio of melamine or urea:formalhyde:alkanol is in the range of 1:(0.1 - 6):(0.1-6). The resulting ether moiety-containing product may by used 'as a cross-linking agent for the aforementioned substituted or un-substituted acrylic acid polymer or copolymer, or it may be self-condensed to form dimers, trimers and/or tetramers which may also be used as cross-linking agents for the aforementioned substituted or un-substituted acrylic acid polymers or co-polymers. Methods for formation of such melamine-formaldehyde and urea-formaldehyde pre-condensates are set forth in U.S. Patent 3,516,846, U.S. Patent 6,261,483, and Lee et al. J. Microencapsulation, 2002, Vol. 19, No.5, pp 559-569, "Microencapsulation of fragrant oil via in situ polymerization: effects of pH and melamine-formaldehyde molar ratio". Examples of urea-formaldehyde pre-condensates useful in the practice of our invention are URAC 180 and URAC 186, Cytec Technology Corp. Examples of melamine-formaldehyde pre-condensates useful in the practice of our invention are CYMEL U-60, CYMEL U-64 and CYMEL U-65, Cytec Technology Corp.

In practicing our invention, the range of mole ratios of urea-formaldehyde precondensate or melamine-formaldehyde pre-condensate:substituted or un-substituted acrylic acid polymer or co-polymer is in the range of from about 9:1 to about 1:9, preferably from about 5:1 to about 1:5 and most preferably from about 1:2 to about 2:1.

The average outside diameter of the resulting microcapsule is in the range of from about 0.01 microns to about 1000 microns; preferably from about 0.05 microns to about 100 microns and more preferably from about 2.0 microns to about 20 microns. The average wall thickness of the resulting microcapsule is in the range of from about 0.001 microns to about 100 microns; preferably from about 0.005 microns to about 10 microns and more preferably from about 0.2 microns to about 2.0 microns.

The content of the resulting microcapsule includes a fragrance composition and/or a benefit agent such as a malodour counteractant composition in combination with a compatible hydrophobic solvent. The term "compatible" is herein intended to mean essentially chemically non-reactive with every fragrance component and/or benefit agent such as a malodour counteractant component and preferably capable of forming a single liquid phase with each fragrance composition component and with each benefit agent component such as a malodour counteractant composition component. In the practice of our invention, the range of weight percent of solvent/fragrance composition components and/or solvent/malodour counteractant composition components contained in each of the microcapsules is from about 5% to about 98%, preferably from about 50 to about 97% by weight of the microcapsule, most preferably from about 91% to about 96%. Thus, the range of weight ratios of encapsulating polymer to solvent/fragrance composition components and/or solvent/malodour counteractant components is preferably from about 1:25 to about 1:1; most preferably from about 1:10 to about 4:96. In addition, the range of weight percent of solvent in the microcapsule is preferably from about 10% to 80% by weight of the filled microcapsule. In a highly preferred ratio of weight of solvent:weight of encapsulated fragrance composition and/or encapsulated malodour counteractant composition is from about 2:1 to about 1:2, with the most preferred ratio being 1:1.

The compatible hydrophobic solvent used in combination with the microencapsulated fragrance composition and/or microencapsulated benefit agent, e.g., malodour counteractant composition is preferably a mono-, di- or tri-$C_4$-$C_{26}$ saturated or unsaturated fatty acid glyceride, diethyl phthalate, dibutyl phthalate, diisodecyl adipate, a liquid polydimethyl siloxane, a liquid polydimethylcyclosiloxane, the methyl ester of soya fatty acid, a mixture of soya fatty acid methyl ester and isopropyl myristate with the weight ratio of soya fatty acid:isopropyl myristate being from 2:1 to 20:1 and a mineral oil compatible with each component of said fragrance composition and/or said benefit agent, e.g., malodour counteractant composition. More preferably, the solvent is a tri-$C_4$-$C_{26}$ saturated or unsaturated fatty acid glyceride. Most preferably, the solvent is the triglyceride ester of a mixture of caprylic acid and capric acid, commercially available as NEOBEE M-5, Stepan Chemical Company. The C $\log_{10}$P of the solvent is greater than 3.3, where P is the n-octanol/water partition coefficient of the hydrophobic solvent; preferably greater than about 8 and most preferably greater than about 10.

The C $\log_{10}$P of each component of the encapsulated fragrance composition and/or the encapsulated malodour counteractant composition preferably is in the range of from about 3.3 to about 8, where P is the n-octanol/water partition coefficient of the fragrance component, although relatively low percentages of fragrance components having a lower value of C $\log_{10}$P may be used in conjunction with the components having a C $\log_{10}$P of between 3.3 and 8. In a preferred embodiment the fragrance or benefit agent is free of a solid material, but does not preclude the inclusion of crystals, particles and the like.

The performance of the capsules of the present invention may be improved through the use of a vast preponderance of high ClogP fragrance materials. In this embodiment of the invention greater than about 60 weight percent of the fragrance materials have a ClogP of greater than 3.3. In another highly preferred embodiment of the invention more than 80 weight percent of the fragrances have a ClogP value of greater than about 4.0. Use of fragrance materials as described previously reduces the diffusion of fragrance through the capsule wall and into the base under specific time, temperature, and concentration conditions.

The higher ClogP materials are preferred, meaning that those materials with a ClogP value of 4.5 are preferred over those fragrance materials with a ClogP of 4; and those materials are preferred over the fragrance materials with a ClogP of 3.3.

The fragrance formulation of the present invention should have at least about 60 weight percent of materials with ClogP greater than 3.3, preferably greater than about 80 and more preferably greater than about 90 weight percent of materials with ClogP greater than 4.

Those with skill in the art appreciate that fragrance formulations are frequently complex mixtures of many fragrance ingredients. A perfumer commonly has several thousand fragrance chemicals to work from. Those with skill in the art appreciate that the present invention may contain a single ingredient, but it is much more likely that the present invention will comprise at least eight or more fragrance chemicals, more likely to contain twelve or more and often twenty or more fragrance chemicals. The present invention also contemplates the use of complex fragrance formulations containing fifty or more fragrance chemicals, seventy five or more or even a hundred or more fragrance chemicals in a fragrance formulation.

Preferred fragrance materials will have both high ClogP and high vapor pressure.

Para cymene, Caphene, Mandarinal Firm, Vivaldie, Terpinene, Verdox, Fenchyl acetate, Cyclohexyl isovalerate, Manzanate, Myrcene, Herbavert, Isobutyl isobutyrate, Tetrahydrocitral, Ocimene and Caryophyllene.

The values of $C \log_{10}P$ of many functional product ingredients, for example, fragrance ingredients contained in personal treatment compositions and/or cosmetic compositions is discussed in U.S. Patents 5,783,544, 6,528,013, 6.656,923 and 6,652,766. Furthermore, values of $\log_{10}P$ have been reported; for example, the Pomona92 database, available from Daylight Chemical Information Systems, Inc., Daylight CIS, Irvine, California. However, the $\log_{10}P$ values are most conveniently calculated by the "CLOGP" program, also available from Daylight CIS. This program also lists experimental $\log_{10}P$ values when they are available in the Pomona92 database. The "calculated $\log_{10}P$" ($C \log_{10}P$) is determined by the Hansch and Leo "fragment" approach based on the chemical structure of each functional product ingredient, and takes into account the numbers and types of atoms, the atom connectivity and the chemical bonding. The $C \log_{10}P$ values which are the most reliable and widely used estimates for this physicochemical property, are preferably used instead of the experimental $\log_{10}P$ values for the selection of functional ingredients, including perfume ingredients which are useful components in the microencapsulate-containing slurries of our invention.

Specific examples of preferred fragrance components useful in the aminoplast microencapsulates used in the composition and process of our invention, and the molecular weights and $Clog_{10}P$ values of each of said components are set forth in Table II as follows:

Table II

| Fragance Component | Clog$_{10}$P value | Molecular Weight |
|---|---|---|
| amyl salicylate | 4.601 | 208.26 |
| benzyl salicylate | 4.383 | 228.25 |
| β-caryophyllene | 6.333 | 204.36 |
| ethyl undecylenate | 4.888 | 212.34 |
| geranyl anthranilate | 4.216 | 273.38 |
| α-irone | 3.820 | 206.33 |
| β-phenyl ethyl benzoate | 4.058 | 226.28 |
| α-santalol | 3.800 | 220.36 |
| amyl salicylate | 4.601 | 208.26 |
| β-caryophyllene | 6.333 | 204.36 |
| cedrol | 4.530 | 222.37 |
| cedryl acetate | 5.436 | 264.41 |
| cedryl formate | 5.070 | 238.37 |
| cyclohexyl salicylate | 5.265 | 220.29 |
| γ-dodecalactone | 4.359 | 198.31 |
| β-phenylethyl phenyl acetate | 3.767 | 240.31 |

Table II   (continued)

| Fragance Component | Clog$_{10}$P value | Molecular Weight |
|---|---|---|
| 5-acetyl-1,1,2,3,3,6-hexamethyl indane | 5.977 | 258.41 |
| cyclopentadecanolide | 6.246 | 240.39 |
| amyl cinnamic aldehyde | 4.324 | 202.30 |
| linalyl benzoate | 5.233 | 258.36 |

Specific examples of malodour counteractant composition components useful in the aminoplast microencapsulates used in the composition and process of our invention are as follows:

Malodour Counteractant Component Group I:

1-cyclohexylethan-1-yl butyrate;
1-cyclohexylethan-1-yl acetate;
1-cyclohexylethan-1-ol;
1-(4'-methylethyl)cyclohexylethan-1-yl propionate; and
2'-hydroxy-1'-ethyl(2-phenoxy)acetate

each of which compound is marketed under the trademark VEILEX® by International Flavors & Fragrances Inc., New York, N.Y.

Malodour Counteractant Component Group II, as disclosed in U.S. Patent 6,379,658:

β-naphthyl methyl ether;
β-naphthyl ketone;
benzyl acetone;
mixture of hexahydro-4,7-methanoinden-5-yl propionate and hexahydro-4,7-methanoinden-6-yl propionate;
4-(2,6,6-trimethyl-2-cyclohexen-1-yl)-3-methyl-3-buten-2-one;
3,7-dimethyl-2,6-nonadien-1-nitrile;
dodecahydro-3a,6,6,9a-tetramethylnaphtho(2,1-b)furan;
ethylene glycol cyclic ester of n-dodecanedioic acid;
1-cyclohexadecen-6-one;
1-cycloheptadecen-10-one; and
corn mint oil.

Insect repellent agents useful in the practice of our invention are disclosed in Published Application for U.S. Patent 2003/0005522 A1 published on January 9, 2003. Preferred insect repellent components useful in the practice of our invention are geraniol, geranium oil, citral and nerol.

Optionally, in order to provide an increased period of time during which the microencapsulates are retained on surfaces to be treated using the consumable products into which the suspensions of our invention are incorporated, the aminoplast microencapsulates used in the practice of our invention may be coated with a cationic polymer as disclosed in Application for U.S. Letters Patent Serial Number 10/718,240 filed on November 20, 2003 and, in addition, Applications for U.S. Patent 10/268,566 and 10/268,526 filed on October 10, 2002. The rate of use of such cationic polymer coatings on the microencapsulates is from about 1% to about 3000% by weight of the filled microencapsulates; preferably from about 5% to about 1000% by weight of the filled microencapsulates; and most preferably from about 10% to about 500% by weight of the filled microencapsulates.

Examples of such cationic polymers used as coatings are cationically modified starch and cationically modified guar, polymers comprising poly diallyl dimethyl ammonium halides (PolyDADMAC), and copolymers of DADMAC with vinyl pyrrolidone, acrylamides, imidazoles, imidazolinium halides, and the like. For instance, Polyquaternium-6, 7, 22 and 39, all available from Ondeo Nalco.

The preferred cationic starch has a molecular weight of from about 100,000 to about 500,000,000, preferably from about 200,000 to about 10,000,000 and most preferably from about 250,000 to about 5,000,000. The preferred cationic starch products are HI-CAT CWS42 and HI-CAT 02 and are commercially available from ROQUETTE AMERICA, Inc.

The preferred cationic guar has a molecular weight of from about 50,000 to about 5,000,000. The preferred cationic guar products are Jaguar C-162 and Jaguar C-17 and are commercially available from Rhodia Inc.

Additional examples of cationic polymers useful for coating the aminoplast encapsulated solvent/fragrance compositions and/or solvent/malodour counteractant compositions of our invention are the water-soluble cationic amino resins, cationic urea resins, specifically, urea-formaldehyde pre-polymers subjected to polycondensation with a cationic modifier such as diethylenetriamine, tetraethylene pentamine, guanidine, guanyl urea and oxazolidine as disclosed in published U.S. Patent Application 2001/008874 A1 published on July 19,2001, for example, U-RAMIN P-1500, Mitsui Kagaku K.K., a urea-formaldehyde pre-polymer modified with diethylene triamine.

An additional embodiment of the invention includes a stable suspension of microencapsulated fragrances in an oil-in-water emulsion where the capsule wall is relatively permeable. The details of such microencapsulated fragrances are set forth in co-pending application for U.S. Letters Patent Serial Number 10/718,240 filed on November 20, 2003. In such a case, since the capsule wall is permeable, it is possible for capsules containing a core of hydrophobic or high C $\log_{10}$ P fragrance materials optionally in combination with one or more high C $\log_{10}$ P compatible solvents, to actually absorb fragrance materials from a fragrance containing base, e.g., a fragranced fabric conditioner/softener base such as that described in U.S. Patent 5,411,671. This process can be improved via the initial inclusion of a more soluble solvent, which may be a lower C $\log_{10}$ P material, in the core which partitions out of the core when placed in the base, thus providing free volume for fragrance material initially present in the base to occupy.

The migration of fragrance materials into the capsule also provides for the production of capsules by simply loading the capsules into a high concentration of fragrance material. The fragrance materials will preferably migrate into the core of the capsules. This allows an encapsulated fragrance to be manufactured by the selection of a permeable capsule material and hydrophobic core and immersing the capsules in a liquid system that contains a high fragrance loading.

In such a case, each of the microcapsules is a permeable microcapsule containing at least 20 weight percent of a sacrificial solvent capable of migrating outside of the capsule over a period of time ranging from about 50 hours to about 200 hours. Preferable sacrificial solvents are benzyl acetate and n-octanol or mixtures thereof, e.g., a 40:60 wt.:wt. mixture of benzyl acetate:n-octanol.

Accordingly, an additional embodiment of our invention is the above-defined stable suspension wherein each of the microcapsules contains the second fragrance composition in admixture with a hydrophobic solvent composition and is prepared according to a process comprising the steps of:

(i) providing a product base containing the non-confined first fragrance composition and the anionic, zwitterionic and/or non-ionic emulsifier material;
(ii) providing a permeable capsule material wherein the permeable capsule material contains greater than about 70 weight percent of the second fragrance composition optionally in combination with a compatible high C $\log_{10}$ P solvent having a C $\log_{10}$ P value of greater than about 3.3; and
(iii) allowing the non-confined first fragrance composition and the permeable capsule material containing the second fragrance composition to come to equilibrium thereby transporting a portion of the non-confined first fragrance composition through the permeable shell wall into the interior of the capsule and retaining the fragrance contents of the permeable capsule.

More specifically an embodiment of our invention is directed to a stable suspension as defined supra wherein each of the microcapsules is produced according to a process comprising the steps of:

(i) providing a sacrificial solvent having a C $\log_{10}$ P value of from about 1 to about 3;
(ii) encapsulating the sacrificial solvent with a permeable encapsulate material;
(iii) providing the encapsulated sacrificial solvent in an liquid environment containing high C $\log_{10}$ P fragrance components with C $\log_{10}$ P of greater than about 3.3; and
(iv) allowing the capsules containing the sacrificial solvent to come to equilibrium with the environment containing the high C $\log_{10}$ P fragrance components;

whereby at least 20 weight percent of the sacrificial solvent migrates from the capsule into the environment.

## The Non-Confined Fragrance and/or Benefit Agent Composition

The non-confined fragrance and/or benefit agent composition in the stable suspension of our invention is contained in the "oil-in-water" emulsion droplets which are part of the emulsion in which the microencapsulated fragrance and/or benefit agent is suspended. The C $\log_{10}$P range of each of the non-confined fragrance and/or benefit agent components is in the range of from about 1 to about 15 thus enabling a greater range of fragrance and/or benefit agent component types in the non-confined fragrance and/or benefit agent as opposed to the components of the 'confined' or 'microencapsulated' fragrance and/or benefit agent.

Within the scope of our invention, each of the oil phase component droplets of the emulsion containing non-confined fragrance and/or benefit agent has a diameter in the range of from about 0.01 to about 10 microns; preferably in the range of from about 0.05 to about 0.8 microns, and more preferably in the range of from about 0.1 to about 0.5 microns.

Specific examples of non-confined fragrance components, their molecular weights and their C $\log_{10}P$'s are set forth in the following Table III:

Table III

| Fragance Component | Clog$_{10}$P value | Molecular Weight |
|---|---|---|
| benzaldehyde | 1.480 | 106.12 |
| benzyl acetate | 1.960 | 150.17 |
| laevo-carvone | 2.083 | 150.22 |
| geraniol | 2.649 | 154.26 |
| cis-jasmone | 2.712 | 164.25 |
| β-phenylethyl alcohol | 1.183 | 122.17 |
| α-terpineol | 2.569 | 154.25 |
| 1-phenyl hexanol-5 | 3.299 | 178.28 |
| dihydromyrcenol | 3.03 | 156.27 |
| δ-undecalactone | 3.830 | 184.28 |
| amyl cinnamate | 3.771 | 218.30 |
| benzophenone | 3.120 | 182.22 |
| nerol | 2.649 | 154.25 |
| 2-methoxynaphthalene | 3.235 | 158.20 |
| ethyl undecylenate | 4.888 | 212.34 |
| geranyl anthranilate | 4.216 | 273.38 |
| α-irone | 3.820 | 206.33 |
| α-santalol | 3.800 | 220.36 |
| iso-eugenol | 2.547 | 164.21 |
| amyl salicylate | 4.601 | 208.26 |
| benzyl salicylate | 4.383 | 228.25 |
| β-caryophyllene | 6.333 | 204.36 |
| cedrol | 4.530 | 222.37 |
| cedryl acetate | 5.436 | 264.41 |
| cedryl formate | 5.070 | 238.37 |
| cyclohexyl salicylate | 5.265 | 220.29 |
| γ-dodecalactone | 4.359 | 198.31 |
| ethyl undecylenate | 4.888 | 212.34 |
| geranyl anthranilate | 4.216 | 273.38 |
| β-phenylethyl benzoate | 4.058 | 226.38 |
| β-phenylethyl phenyl acetate | 3.767 | 240.31 |

Table III   (continued)

| Fragance Component | Clog$_{10}$P value | Molecular Weight |
|---|---|---|
| 5-acetyl-1,1,2,3,3,6-hexameth indane | 5.977 | 258.41 |
| cyclopentadecanolide | 6.246 | 240.39 |
| d-limonene | 4.232 | 136.24 |
| cis-p-t-butylcyclohexyl acetat | 4.019 | 198.31 |
| amyl cinnamic aldehyde | 4.324 | 202.30 |

The suspension containing the confined and non-confined fragrance and/or benefit agent may also contain at least one of the following auxiliary substances in amounts of from about 0.01% to about 30% by weight of the non-confined fragrance and/or benefit agent composition:

at least one deposition aid;
at least one additional surfactant;
at least one humectant;
at least one viscosity control agent; and
at least one solvent.

Examples of such auxiliary substances are set forth in co-pending Applications for U.S. Letters Patent Serial Numbers 10/268,566 and 10/268,526 filed on October 10, 2002.

## The Utility of the Suspensions

Our invention is also directed to the use of the aforementioned suspensions of our invention as a fragrance modifier or additive or as a benefit agent, e.g., malodour counteractant or insect repellent, additive to various consumable articles including but not limited to liquid anionic, cationic, non-ionic or zwitterionic detergents, shampoos, bodywashes, soaps, hair conditioners, skin lotions, skin creams, skin moisturizers, anti-perspirants, deodorants and liquid fabric softener and/or fabric conditioner compositions. The following Table IV sets forth specific consumable articles for which the suspensions of our invention are useful and U.S. Patents setting forth the detailed description of said consumable articles:

Table IV

| U.S. Patent Number | Nature of Use and/or Title |
|---|---|
| U.S. Patent 4,515,705 | Heavy duty liquid detergents containing perfumes and proteolytic enzymes |
| U.S. Patent 5,411,671 | Aqueous fabric conditioning compositions |
| U.S. Patent 5,574,179 | Biodegradable quaternary ammonium fabric softener compositions |
| U.S. Patent 5,880,084 | Liquid rinse cycle fabric softening compositions containing iacid polymeric fatty ester quaternary ammonium compounds |
| U.S. Patent 6,620,437 | Perfume-containing water-in-oil microemulsion for providing cosmetic attributes to fabric softening base composition |
| U.S. Patent 6,620,777 | Fabric care composition comprising cationic softening compound, non-confined fragrance oil, encapsulated fragrance oil and water |
| U.S. Patent 6,664,223 | Fabric care composition containing polycarboxylate polymer, urea derivative, e.g., a hydroxyalkylurea, water and a surfactant |
| U.S. Patent 6,693,065 | A non-foaming detergent composition for concentrated alkaline media |
| U.S. Patent 6,693,068 | Alkaline carpet cleaning composition comprising a pyrrolidone-based solvent |
| U.S. Patent 6,696,053 | Leave-on or rinse-out hair care conditioner compositions containing silicone quaternary compounds and thickeners |

Table IV   (continued)

| U.S. Patent Number | Nature of Use and/or Title |
|---|---|
| U.S. Patent 6,696,395 | Perfumed liquid household cleaning and deodorizing compositions |
| U.S. Patent 6,696,402 | Laundry detergent compositions containing zwitterionic polyamines |
| U.S. Patent 6,699,824 | Cleansing compositions comprising highly branched poly-$\alpha$-olefins |

**BRIEF DESCRIPTION OF THE DRAWINGS**

Figure 1 is a perspective view, on a greatly enlarged scale of a fragrance and/or malodour counteractant-containing microcapsule useful in the practice of our invention.

Figure 2 is a cross-sectional view of a first embodiment of the microcapsule of Figure 1.

Figure 3 is a cross-sectional view of a second embodiment of the microcapsule of Figure 1 being coated with a cationic polymer coating.

Figure 4A is a cross-sectional view of a third embodiment of the microcapsule of Figure 1, showing in schematic form, a first mechanism for formation of the microcapsule wall.

Figure 4B is a cross-sectional view of a third embodiment of the microcapsule of Figure 1, showing in schematic form, a second mechanism for formation of the microcapsule wall.

Figure 5A is a cut-away side elevation view of the suspension of our invention containing the microcapsules of Figure 2 suspended in an oil-in-water emulsion wherein droplets containing non-confined fragrance and/or benefit agent are suspended in water.

Figure 5B is a cut-away side elevation view of the suspension of our invention containing the cationic polymer-coated microcapsules of Figure 3 suspended in an oil-in-water emulsion wherein droplets containing non-confined fragrance and/or benefit agent are suspended in water.

Figure 5C is a differential volume distribution diagram for the suspended microcapsules and for the oil-in-water emulsion droplets as illustrated in Figure 5A indicating on the "X" axis, particle diameter measured in units of microns (for the microcapsules suspended in the emulsion) and droplet diameter measured in units of microns (for the oil phase non-confined fragrance and/or benefit agent emulsion droplets suspended in the water) vs. volume % of microcapsule or emulsion droplet as measured along the "Y" axis.

Figure 5D is a graph of viscosity (in centipoises at a shear rate of 1.0 seconds$^{-1}$ and at 25°C )(measured along the "Y" axis) of emulsifier-free suspension of microencapsulated fragrance in a capsule slurry suspension vs. storage time (in days) measured along the "X' axis for four suspensions each containing a different fragrance; that of Example A; that of Example B; that of Example C; and that of Example D, each of which Example is set forth. The graph has 52 data pairs.

Figure 5E is a graph containing the 52 data pairs of Figure 5D wherein emulsifier-free suspension storage time (measured in days along the "X" axis) is plotted against $10(\log_{10}$(emulsifier-free suspension viscosity)-3) (measured along the "Y" axis) showing a 'best-fit' regression function defined according to the algorithm:

$$10(\log_{10}\nu\text{-}3) = 0.28\theta + 3.05$$

with a standard error of estimate of 2.94, which algorithm corresponds to the algorithm set:
$\log_{e}\nu = 0.065\theta + 7.62$; and

$$\frac{\partial \nu}{\partial \theta} = 0.065\nu$$

wherein "$v$" is viscosity measured in centipoises at a shear rate of 1.0 seconds[-1] and at 25°C and "$\theta$" is storage time measured in days.

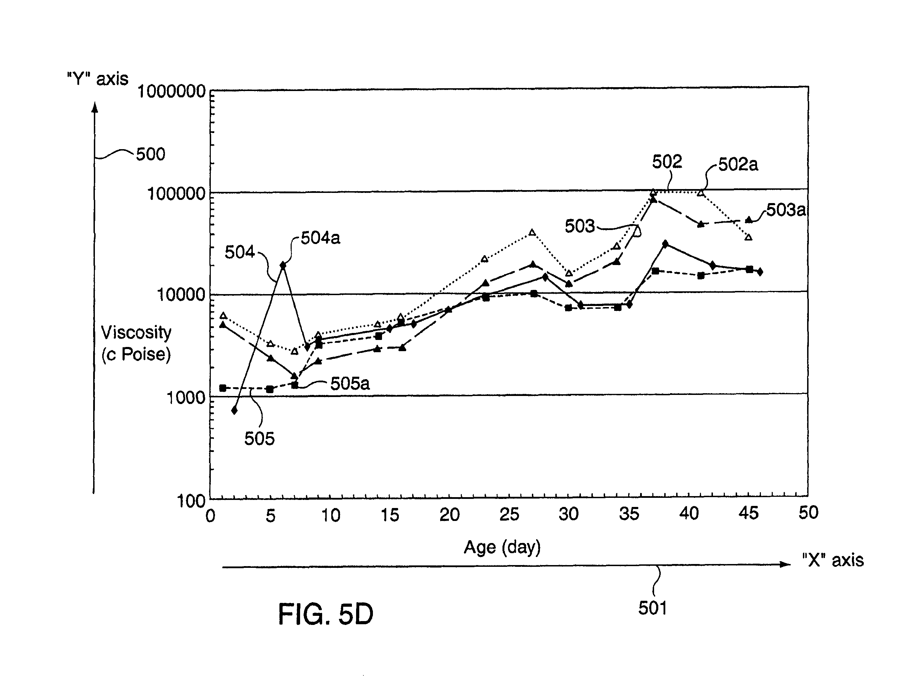

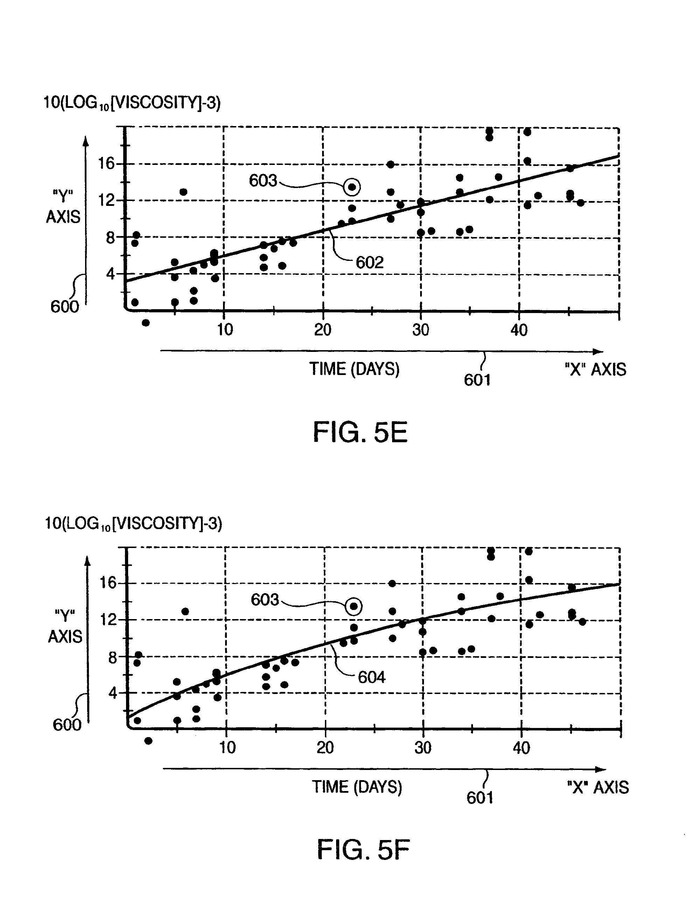

Figure 5F is a graph containing the 52 data pairs of Figure 5D wherein emulsifier-free suspension storage time (measured in days along the "X" axis) is plotted against 10(log$_{10}$(emulsifier-free suspension viscosity)-3) (measured along the "Y" axis) showing a 'best-fit' regression function defined according to the algorithm:

$$0(\log_{10}v\text{-}3) = \text{-}19.4e^{\text{-}0.028\theta} + 20.5$$

with a standard error of estimate of 2.97, which algorithm corresponds to the algorithm set:

$$\log_e v = \text{-}4.47e^{\text{-}0.028\theta} + 11.64;$$

and

$$\frac{\partial v}{\partial \theta} = 0.125v e\text{-}^{0.028\theta}$$

wherein "$v$" is viscosity measured in centipoises at a shear rate of 1.0 seconds[-1] and at 25°C and "$\theta$" is storage time measured in days.

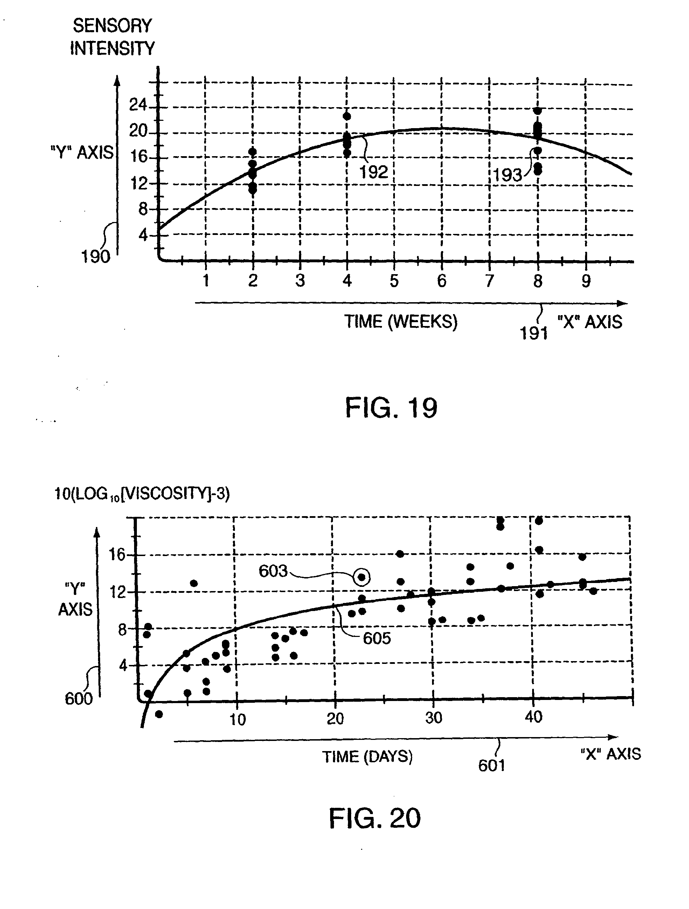

Figure 20 is a graph containing the 52 data pairs of Figure 5D wherein emulsifier-free suspension storage time (measured in days along the "X" axis) is plotted against 10(log$_{10}$(emulsifier-free suspension viscosity)-3) (measured along the "Y" axis) showing a 'best-fit' regression function defined according to the algorithm:

$$10(\log_{10}v\text{-}3) = 3.44 \log_e\theta - 0.234$$

with a standard error of estimate of 2.97, which algorithm corresponds to the algorithm set:

$$\log_e v = 0.793 \log_e\theta + 6.86;$$

and

$$\frac{\partial v}{\partial \theta} = 0.793\left(\frac{v}{\theta}\right)$$

wherein "$v$" is viscosity measured in centipoises at a shear rate of 1.0 seconds[-1] and at 25°C and "0" is storage time measured in days.

Figure 5G is a graph of viscosity (in centipoises at a shear rate of 1.0 seconds[-1] and at 25°C )(measured along the "Y" axis) of emulsifier-containing suspension (containing 2.5% TWEEN 20 non-ionic emulsifier) of microencapsulated fragrance in a non-confined fragrance-water oil-in-water emulsion vs. storage time (in days) measured along the "X' axis for a suspension containing the fragrance of Example A. The graph has 16 data pairs. TWEEN is a registered trademark of ICI Americas Inc. of Bridgewater, N.J.

Figure 5H is a graph containing the 16 data pairs of Figure 5G wherein emulsifier-containing suspension storage time (measured in days along the "X" axis) is plotted against 10(log$_{10}$(emulsifier-containing suspension viscosity)-3) (measured along the "Y" axis) showing a 'best-fit' regression function defined according to the algorithm:

$$10(\log_{10}v\text{-}3) = 0.019\theta + 7.62$$

with a standard error of estimate of 1.17, which algorithm corresponds to the algorithm set:

$$\log_e \nu = 0.0044\theta + 8.67;$$

and

$$\frac{\partial \nu}{\partial \theta} = 0.0044\nu$$

wherein "$\nu$" is viscosity measured in centipoises at a shear rate of 1.0 seconds[-1] and at 25°C and "$\theta$" is storage time measured in days.

Figure 5I is a graph containing the 16 data pairs of Figure 5G wherein emulsifier-containing suspension storage time (measured in days along the "X" axis) is plotted against $10(\log_{10}$(emulsifier-containing suspension viscosity)-3) (measured along the "Y" axis) showing a 'best-fit' regression function defined according to the algorithm:

$$10(\log_{10}\nu - 3) = 7.44e^{0.0024\theta}$$

with a standard error of estimate of 1.20, which algorithm corresponds to the algorithm set:

$$\log_e \nu = 1.71e^{0.0024\theta} + 6.91;$$

and

$$\frac{\partial \nu}{\partial \theta} = 0.0041\nu e^{0.0024\theta}$$

wherein "$\nu$" is viscosity measured in centipoises at a shear rate of 1.0 seconds[-1] and at 25°C and "$\theta$" is storage time measured in days.

Figure 5J is a graph containing the 16 data pairs of Figure 5G wherein emulsifier-containing suspension storage time (measured in days along the "X" axis) is plotted against $10(\log_{10}$(emulsifier-containing suspension viscosity)-3) (measured along the "Y" axis) showing a 'best-fit' regression function defined according to the algorithm:

$$10(\log_{10}\nu - 3) = 0.9 \log_e \theta + 5.55$$

with a standard error of estimate of 0.78, which algorithm corresponds to the algorithm set:

$$\log_e \nu = 0.21 \log_e \theta + 8.19;$$

and

$$\frac{\partial \nu}{\partial \theta} = 0.21\left(\frac{\nu}{\theta}\right)$$

wherein "$\nu$" is viscosity measured in centipoises at a shear rate of 1.0 seconds[-1] and at 25°C and "$\theta$" is storage time measured in days.

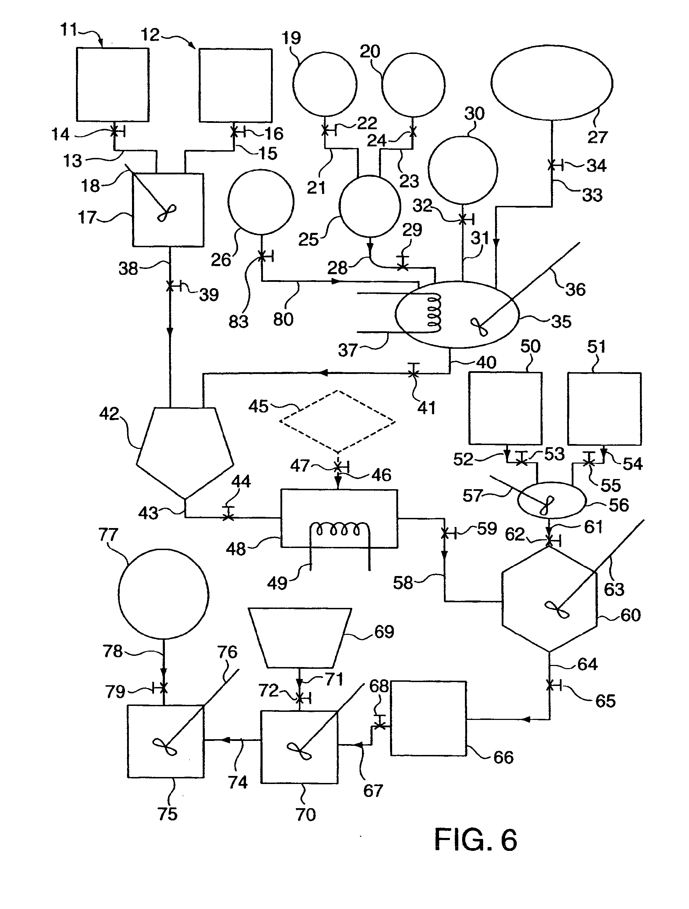

Figure 6 is a schematic block flow diagram setting forth the process steps and apparatus means for formation of the stable initial impact and continuous impact fragrance and/or benefit agent-imparting microcapsule suspension of our invention.

Figure 7A is a schematic perspective view of the first stage of the operation of a rotor/stator high shear mixer used in the process and apparatus of our invention which mixer is indicated by reference numeral 42 of Figure 6, wherein the high speed rotation of the rotor blades within the precision machined mixing workhead exerts a powerful suction drawing the liquid fragrance and/or benefit agent and hydrophobic solvent together with the water and uncured acrylic acid polymer or co-polymer cross-linked with a melamine-formaldehyde pre-condensate or a urea-formaldehyde pre-condensate into the rotor/stator assembly.

Figure 7B is a schematic perspective diagram of stage two of the operation of a rotor/stator high shear mixer used in the processes and apparatus of our invention which mixer is indicated by reference numeral 42 of Figure 6, wherein centrifugal force drives materials towards the periphery of the workhead where they are subjected to a milling action in the precision machined clearance between the ends of the rotor blades and the inner wall of the stator.

Figure 7C is a schematic perspective diagram of the operation of the third stage of a rotor/stator high shear mixer useful in the process and the apparatus of our invention which mixer is indicated by reference numeral 42 of Figure 6, wherein the second stage is followed by intense hydraulic shear as the material is forced, at high velocity, out through the perforations in the stator, then through the machine outlet and along the pipework; while at the same time, fresh materials are continually drawn into the workhead, maintaining the mixing and pumping cycle.

Figure 7D is a schematic side view of the homogenizing equipment assembly for carrying out the blending step of the process of our invention and as part of the apparatus of our invention at the location indicated by reference numeral 42 in Figure 6.

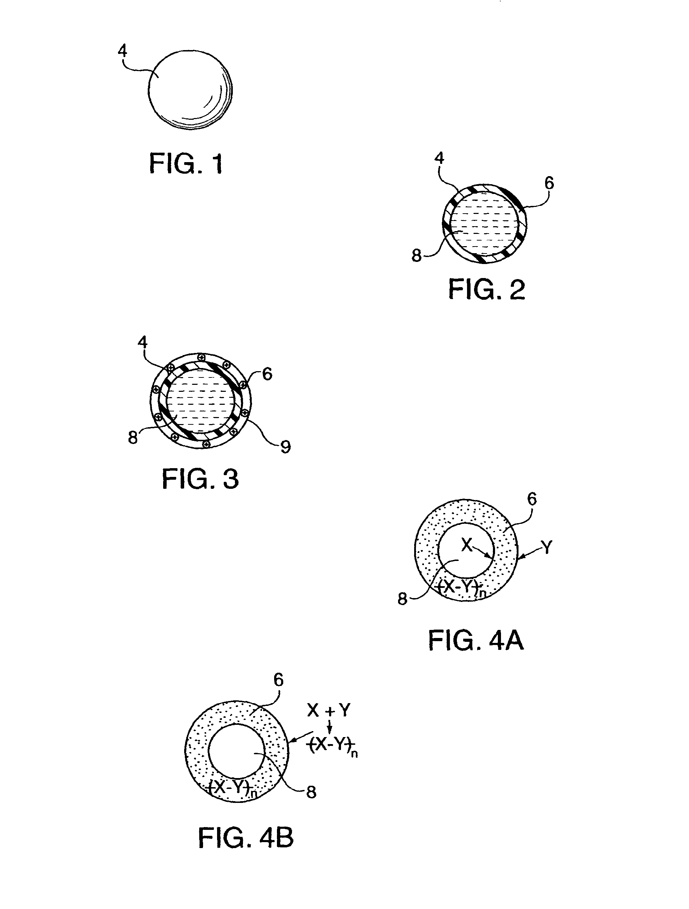

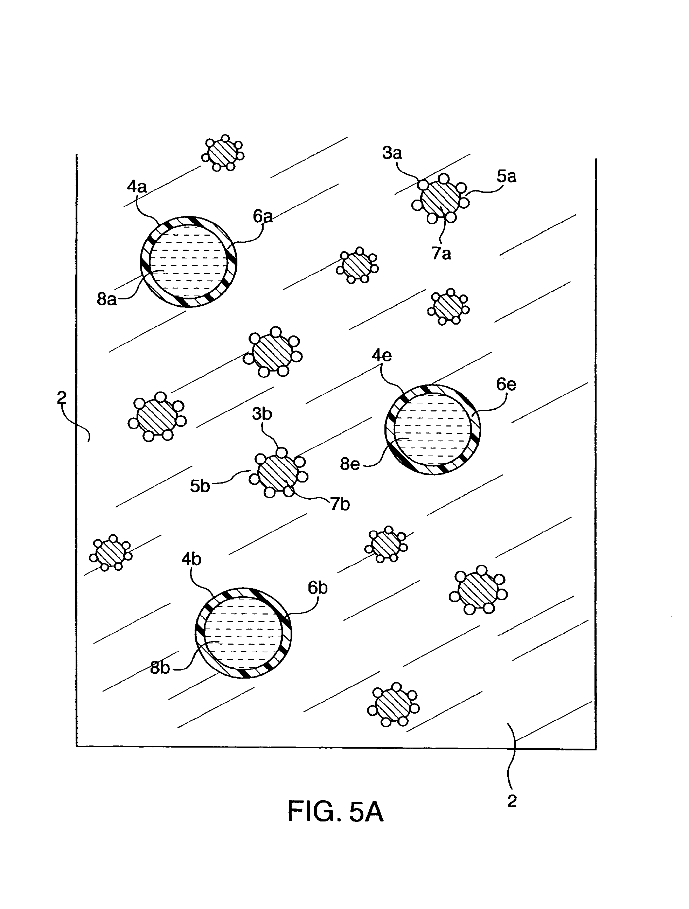

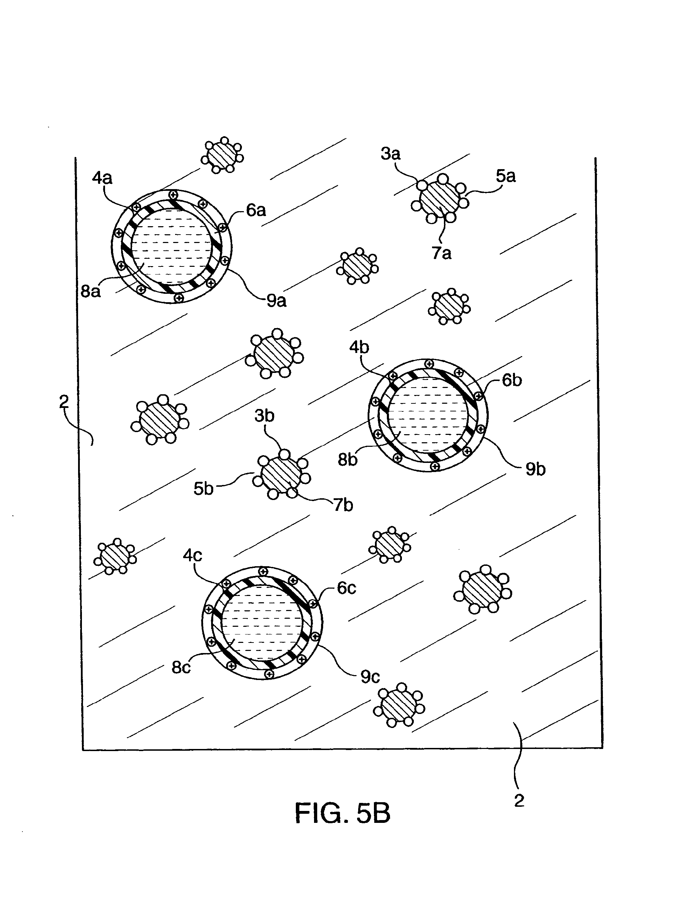

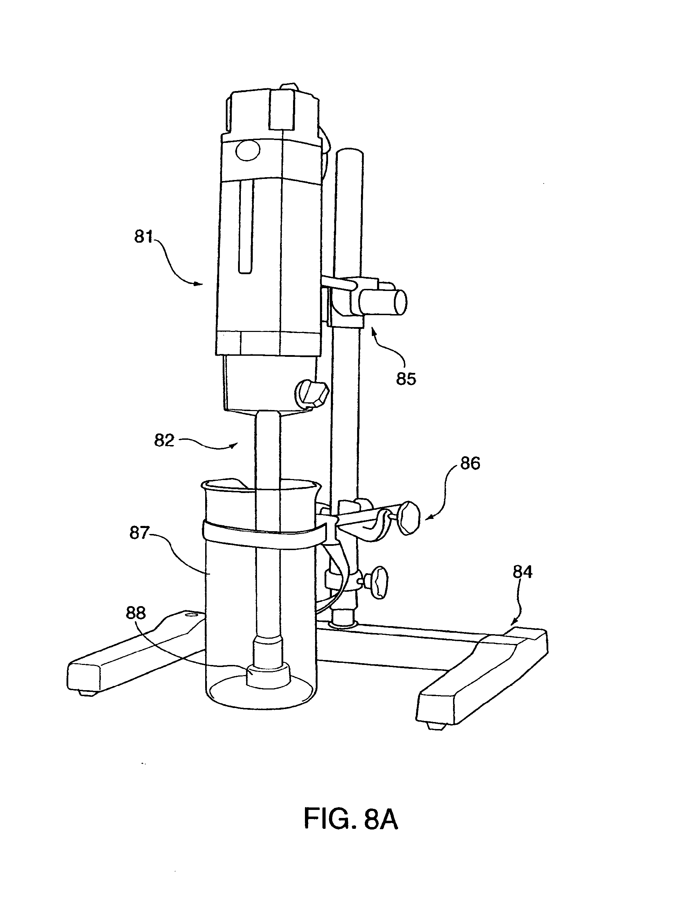

Figure 8A is a schematic perspective view of high shear mixing apparatus indicated by reference numeral 60 of Figure 6, for carrying out the step of the process of our invention for mixing the emulsifier/non-confined fragrance and/or benefit agent composition with the cured fragrance and/or benefit agent-containing microcapsule/water mixture in order to form the stable suspension of our invention shown schematically in Figure 5A or Figure 5B containing the microcapsules of Figure 2 or Figure 3 suspended in an oil-in-water emulsion wherein droplets containing non-confined fragrance and/or benefit agent are suspended in water.

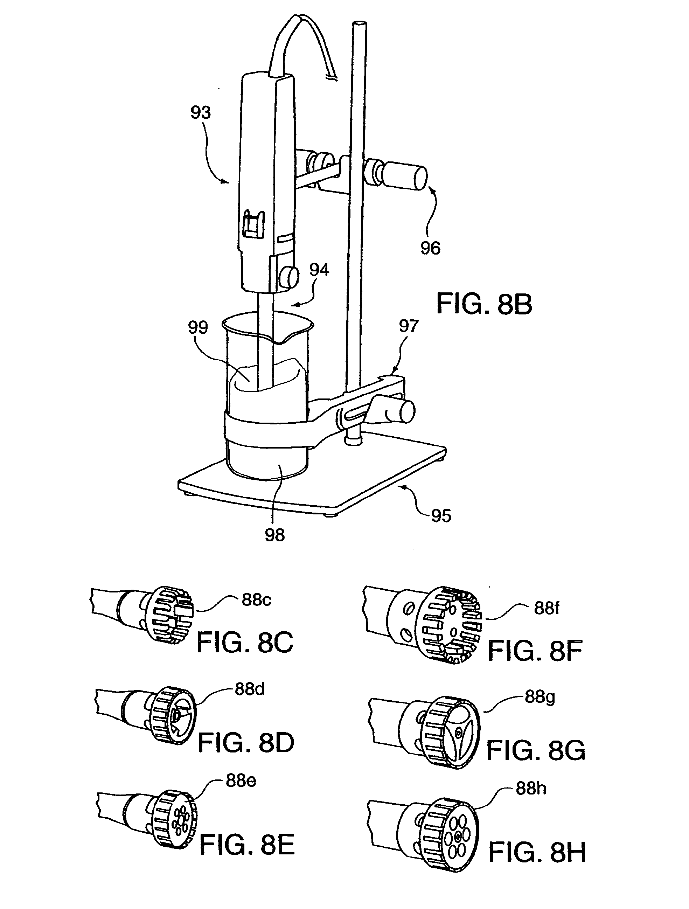

Figure 8B is a schematic view of the high shear mixing apparatus of Figure 8A in operation by effecting the step of the process of our invention for mixing the emulsifier/non-confined fragrance and/or benefit agent composition with the cured fragrance and/or benefit agent-containing microcapsule/water mixture in order to form the stable suspension of our invention.
Each of Figures 8C, 8D, 8E, 8F, 8G and 8H represents a schematic perspective view of a dispersing element (indicated by reference numeral 88 in Figure 8A) for use with the high shear mixing apparatus of Figure 8A which enables the apparatus to be effective for the purpose of forming the stable suspension of our invention.

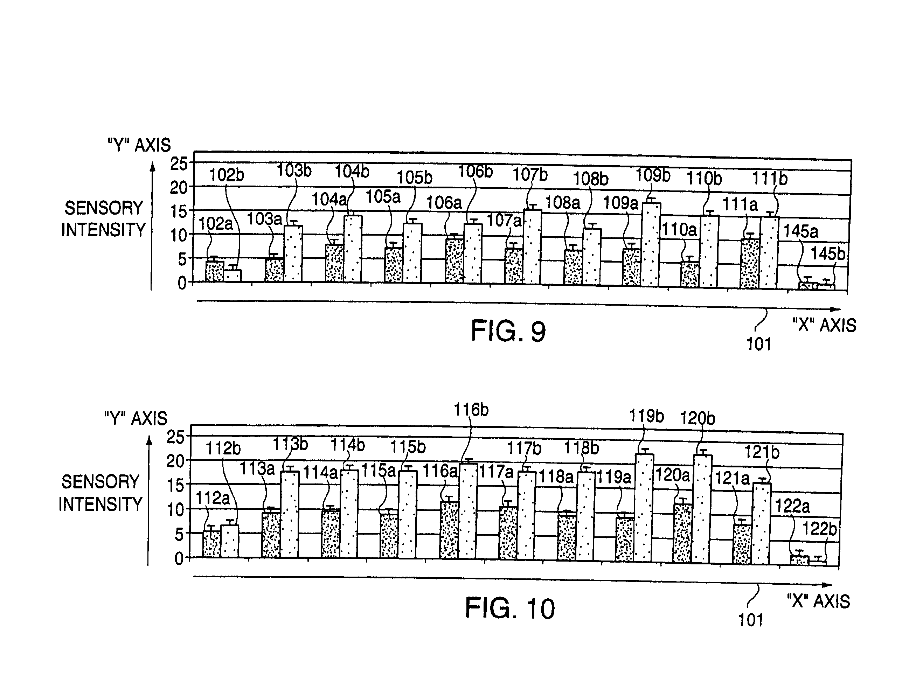

Figure 9 is a set of bar graphs of perceived sensory intensity (on a scale of 0-99 as measured on the "Y" axis) for "pre-rub" (immediately after application of the suspension to fabric swatches, but before rubbing) and "post-rub" (immediately after rubbing the fabric surface to which the suspension-containing base is applied) for (a) fragrance-containing suspensions of our invention each containing either anionic, non-ionic or zwitterionic emulsifiers at a point in time 2 weeks after storage of the suspension at temperatures of 25°C or 37°C wherein the suspension is, immediately subsequent to the 2 week storage period, admixed with a model cationic fabric softener/conditioner base ) and then applied to fabric swatches or (b) neat fragrances tested in the same manner as the suspensions.

Figure 10 is a set of bar graphs of perceived sensory intensity (on a scale of 0-99 as measured on the "Y" axis) for "pre-rub" (immediately after application of the suspension to fabric swatches, but before rubbing) and "post-rub" (immediately after rubbing the fabric surface to which the suspension-containing base is applied) for (a) fragrance-containing suspensions of our invention each containing either anionic, non-ionic or zwitterionic emulsifiers at a point in time 4 weeks after storage of the suspension at temperatures of 25°C or 37°C wherein the suspension is, immediately subsequent to the 4 week storage period, admixed with a model cationic fabric softener/conditioner base,) and then applied to fabric swatches or (b) neat fragrances tested in the same manner as the suspensions.

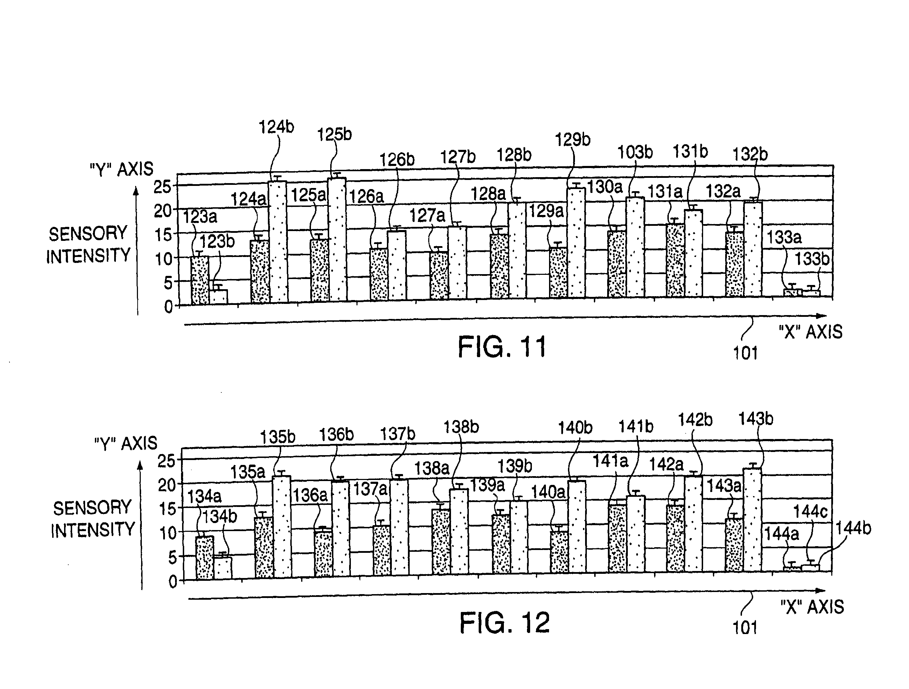

Figure 11 is a set of bar graphs of perceived sensory intensity (on a scale of 0-99 as measured on the "Y" axis) for "pre-rub" (immediately after application of the suspension to fabric swatches, but before rubbing) and "post-rub" (immediately after rubbing the fabric surface to which the suspension-containing base is applied) for (a) fragrance-containing suspensions of our invention each containing either non-ionic or zwitterionic emulsifiers at a point in time 8 weeks after storage of the suspension at temperatures of 25°C or 37°C wherein the suspension is, immediately subsequent to the 8 week storage period, admixed with a model cationic fabric softener/conditioner base) and then applied to fabric swatches or (b) neat fragrances tested in the same manner as the suspensions.

Figure 12 is a set of bar graphs of perceived sensory intensity (on a scale of 0-99 as measured on the "Y" axis) for "pre-rub" (immediately after application of the suspension to fabric swatches, but before rubbing) and "post-rub" (immediately after rubbing the fabric surface to which the suspension-containing base is applied) for (a) fragrance-containing suspensions of our invention each containing either anionic, non-ionic or zwitterionic emulsifiers at a point in time 2 weeks after storage of the suspension at a temperature of 37°C wherein the suspension is, prior to the 2 week storage period, admixed with a model cationic fabric softener/conditioner base, - with the model cationic fabric conditioner base-suspension thus stored for 2 weeks then being applied to fabric swatches immediately subsequent to the 2 week storage period or (b) neat fragrances tested in the same manner as the suspensions.

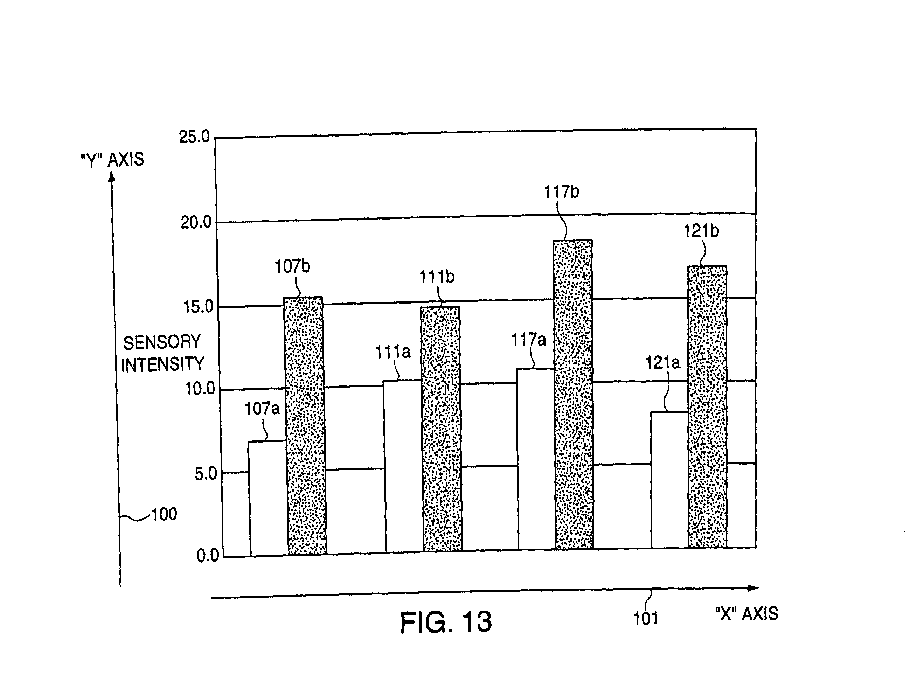

Figure 13 is a set of bar graphs included in Figure 9 and in Figure 10 wherein the emulsifier used in each of the suspensions of our invention is limited to the anionic emulsifier, sodium lauryl sulfate (HLB value = 40).

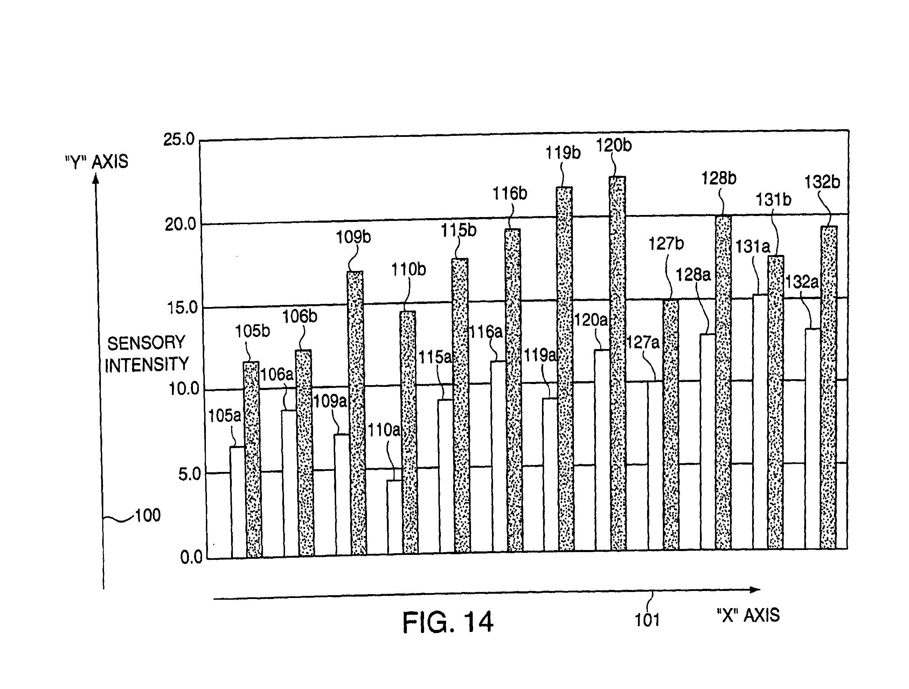

Figure 14 is a set of bar graphs included in each of Figure 9, Figure 10 and Figure 11 wherein the emulsifier used in each of the suspensions of our invention is limited to the non-ionic emulsifier, TWEEN 20 (polyoxyethylene(20) sorbitan monolaurate).

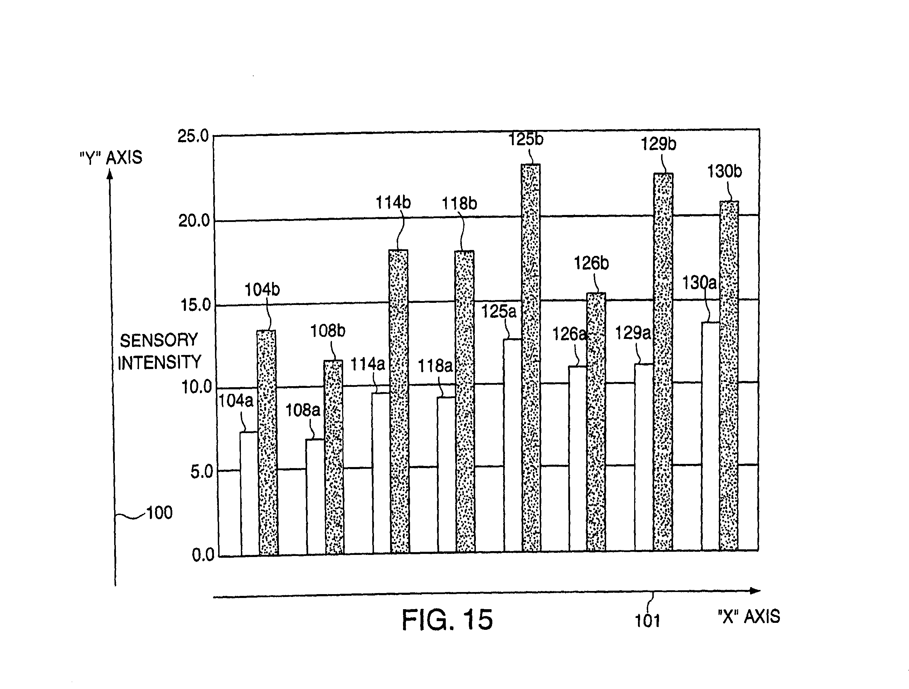

Figure 15 is a set of bar graphs included on each of Figure 9, Figure 10 and Figure 11 wherein the emulsifier used in each of the suspensions of our invention is limited to the zwitterionic emulsifier, the lecithin, CENTROPHASE HR 4B, trademark of the Central Soya Company, Inc. of Fort Wayne, Indiana, a mixture of phosphatidyl cholines, phosphatadylethanolamines and phosphatidylinositols.

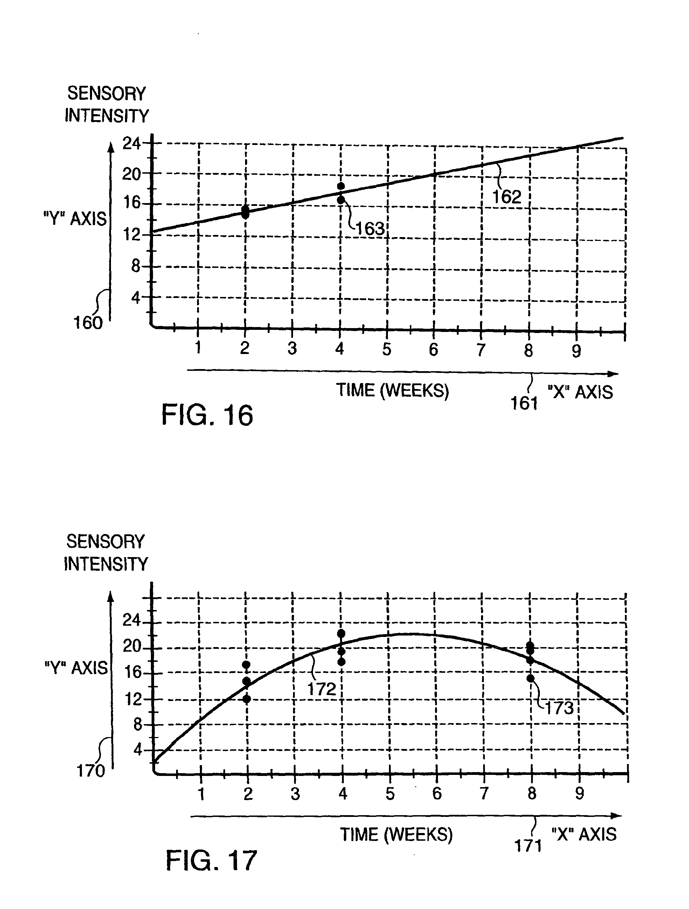

Figure 16 is a graph for the data of Figure 13 with sensory intensity (on a scale of 0-99) on the "Y" axis and time in weeks on the "X" axis. The regression algorithm obtained from the data in Figure 13 is as follows:

$$Y = 1.25X + 12.5$$

with a standard error of estimate = 0.604.

Figure 17 is a graph for the data of Figure 14 with sensory intensity (on a scale of 0-99) on the "Y" axis and time in weeks on the "X" axis. The regression algorithm obtained from the data in Figure 14 is as follows:

$$Y = -0.64X^2 + 7.13X + 2.13$$

with a standard error of estimate = 2.02.

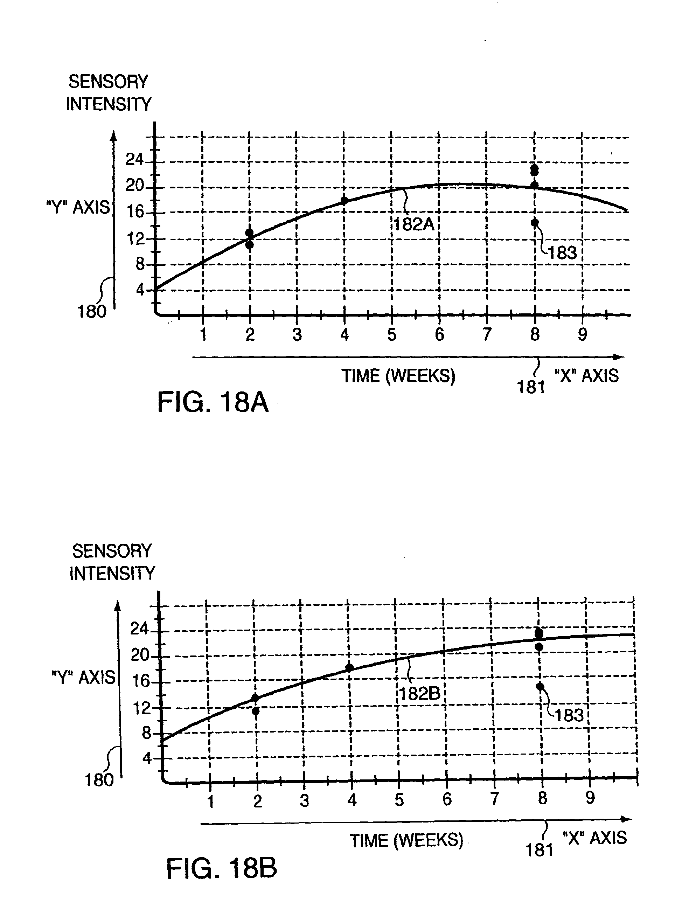

Figures 18A and 18B are separate graphs for the data of Figure 15 with sensory intensity (on a scale of 0-99) on the "Y" axis and time in weeks on the "X" axis. The regression algorithms obtained from the data in Figure 15 are as follows:

$$Y = -0.375X^2 + 5.03X + 3.9$$

with a standard error of estimate = 2.52 and

$$Y = -17.13e^{-0.235X} + 24$$

with a standard error of estimate = 2.73.

Figure 19 is a graph for the combined data of each of Figures 13, 14 and 15 with sensory intensity (on a scale of 0-99) on the "Y" axis and time in weeks on the "X" axis. The regression algorithm obtained from the data in Figures 13, 14 and 15 is as follows:

$$Y = -0.44X^2 + 5.28X + 4.98$$

with a standard error of estimate = 2.33.

## DETAILED DESCRIPTION OF THE DRAWINGS

Each of the microcapsules of Figures 1, 2, 3, 4A and 4B is designated in its entirety by the reference numeral 4. Microcapsule 4 preferably comprises an aminoplast shell 6 having a fill 8 therewith which fill comprises a fragrance composition and/or a malodour counteractant composition in admixture with a solvent as described and exemplified. Figure 3 shows the microcapsule coated with a cationic polymer, with the cationic polymer coating indicated by reference numeral 9. As indicated, such cationic polymers are disclosed in Applications for U.S. Patent 10/268,566 and 10/268,526 filed on October 10, 2002. Figures 4A and 4B indicate, schematically, the formation of the polymeric aminoplast shell by means of interfacial polymerization reaction of a melamine-formaldehyde precondensate, e.g., URAC 180 (shown as "X") with an acrylic acid polymer or copolymer (shown as "Y") thereby forming the cross-linked acrylic acid polymer or copolymer ("-(-X-Y-)$_N$-")- In Figure 4A, the melamine formaldehyde precondensate is initially contained in the solvent-fragrance and/or malodour counteractant phase, 4 and the acrylic acid polymer or co-polymer is dispersed in an aqueous phase. Reaction takes place at location 6 where the cross-linked acrylic acid polymer or copolymer (the 'aminoplast resin') is formed. Curing at higher temperatures, e.g., 50 - 85°C then takes place at location 6. In Figure 4B each of the melamine-formaldehyde precondensate and the acrylic acid polymer or co-polymer is dispersed in the aqueous phase and reacted under acidic conditions. The resulting un-cured cross-linked acrylic acid polymer or co-polymer coacervates about the solvent-fragrance and/or malodour counteractant phase droplets at location 6. Curing at higher temperatures, e.g., 50 - 85°C then takes place at location 6.

In Figure 5A the non-coated microcapsules 4a, 4b and 4c of Figure 2 respectively having walls 6a, 6b and 6c and confined fragrance and/or benefit agent 8a, 8b and 8c are shown to be suspended in an oil-in-water emulsion wherein droplets 5a and 5b containing non-confined fragrance and/or benefit agent, respectively 7a and 7b, and associated with anionic, non-ionic or zwitterionic emulsifer 3a and 3b are stably suspended in water body 2.

In Figure 5B the cationic polymer-coated microcapsules 4a, 4b and 4c of Figure 3 respectively having cationic polymer coatings 9a, 9b and 9c, walls 6a, 6b and 6c and confined fragrance and/or benefit agent 8a, 8b and 8c are shown to be suspended in an oil-in-water emulsion wherein droplets 5a and 5b containing non-confined fragrance and/ or benefit agent, respectively 7a and 7b, and associated with anionic, non-ionic or zwitterionic emulsifier 3a and 3b are stably suspended in water body 2.

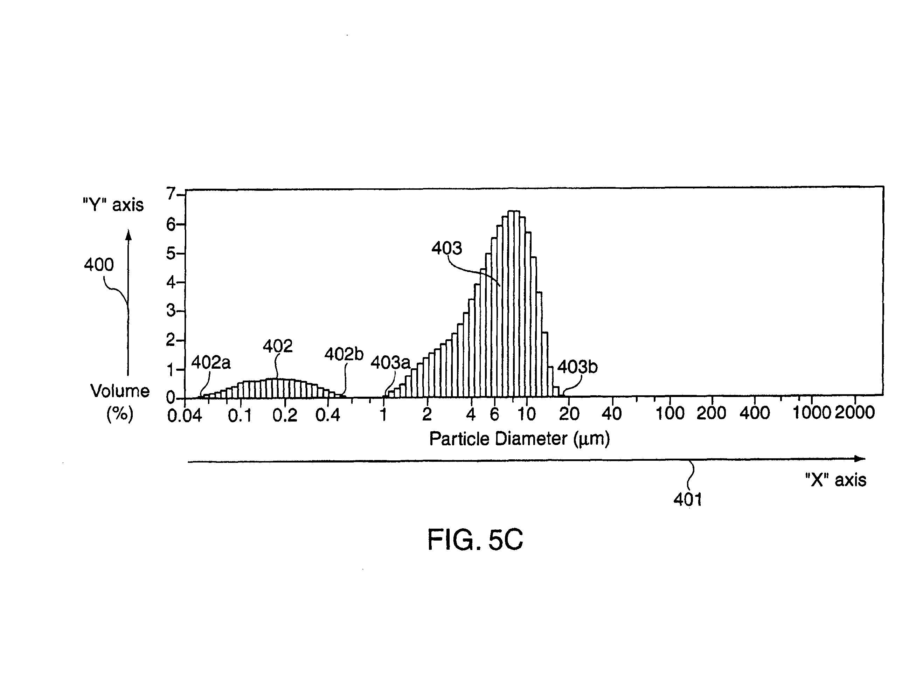

Figure 5C is an illustrative differential volume distribution diagram for the suspended microcapsules 4a, 4b and 4c and for the oil-in-water emulsion droplets 5a and 5b as illustrated in Figure 5A. Thus, in Figure 5C, the particle diameter limits measured in units of microns (for the microcapsules suspended in the emulsion) and droplet diameter limits measured in units of microns (for the oil phase non-confined fragrance and/or benefit agent emulsion droplets suspended in the water indicated by reference numeral 2 in Figure 5A) is measured along the "X" axis, indicated by reference numeral 401 and the volume % of microcapsules having particle diameters which are less than the indicated particle diameter limit or volume % of emulsion droplets which have droplet diameters less than the indicated droplet diameter limit is measured along the "Y" axis indicated by reference numeral 400. The section of the volume distribution diagram relevant to the suspended microcapsules is indicated by reference numeral 403 with the upper bound of particle diameter range being approximately 20 microns (below which are 100% of the microcapsule particles) and indicated by reference numeral 403b and with the lower bound of particle diameter range being approximately 1 micron (above which are 100% of the microcapsule particles, and below which are 0% of the microcapsule particles) and indicated by reference numeral 402a. More specifically with reference to the illustrative differential volume distribution diagram of Figure 5C, 90% of the microcapsule particles have diameters less than 11.21 microns; 75% of the microcapsule particles have diameters less than 8.798 microns; 50% of the microcapsule particles have diameters less than 6.032 microns; 25% of the microcapsule particles have diameters less than 3.356 microns and 10% of the microcapsule particles have diameters less than 1.168 microns. The section of the volume distribution diagram relevant to the suspended oil-in-water emulsion droplets is indicated by reference numeral 402 with the upper bound of the droplet diameter range being about 0.55 microns (below which are 100% of the emulsion droplets) and indicated by reference numeral 402b and with the lower bound of emulsion droplet diameter range being approximately 0.055 microns (above which are 100% of the emulsion droplets and below which are 0% of the emulsion droplets) and indicated by reference numeral 402a.

Referring to Figure 5D, the viscosity (measured in centipoises at a shear rate of 1.0 seconds$^{-1}$ and at 25°C) of four emulsifier-free microcapsule suspensions containing, respectively, the four different fragrance formulations of Examples A, B, C and D is measured along the "Y" axis, indicated by reference numeral 500 vs. storage time of suspension (measured in days), measured along the "X" axis indicated by reference numeral 501. The graph for the emulsifier-free suspension containing the fragrance of Example A and including 13 data points is indicated by reference numeral 502, with one of the data points therefor indicated by reference numeral 502a. The graph for the emulsifier-free suspension containing the fragrance of Example B and including 13 data points is indicated by reference numeral 503, with one of the data points therefor indicated by reference numeral 503a. The graph for the emulsifier-free suspension containing the fragrance of Example C and including 13 data points is indicated by reference numeral 504, with one of the data points therefor indicated by reference numeral 504a. The graph for the emulsifier-free suspension containing the fragrance of Example D and including 13 data points is indicated by reference numeral 505, with one of the data points therefor indicated by reference numeral 505a.

Each of the graphs of Figures 5E, Figure 5F and Figure 20 is a plot of emulsifer-free suspension storage time (measured in days along the "X" axis, indicated by reference numeral 601) vs. 10(log$_{10}$(emulsifier-free suspension viscosity)-3) (measured along the "Y" axis, indicated by reference numeral 600) for the entire 52 data pairs for each of the four suspensions which is the subject of Figure 5D. Each of the graphs in Figures 5E, Figure 5F and Figure 20 indicated, respectively, by reference numeral 602 ,by reference numeral 604 and by reference numeral 605 includes each of the 52 data points of Figure 5D, one of which data point is indicated by reference numeral 603.

Referring to Figure 5G, the viscosity (measured in centipoises at a shear rate of 1.0 seconds$^{-1}$ and at 25°C) of emulsifier-containing microencapsulated fragrance-containing aqueous suspension of our invention (containing 2.5% TWEEN 20 non-ionic emulsifier ) containing the fragrance formulation of Example A is measured along the "Y" axis, indicated by reference numeral 700 vs. storage time of suspension (measured in days), measured along the "X" axis indicated by reference numeral 701. The graph for the emulsifier-containing microcapsule suspension containing the fragrance composition of Example A and including 16 data pairs is indicated by reference numeral 702, with one of the data points therefor indicated by reference numeral 702a.

Each of the graphs of Figures 5H, Figure 5I and Figure 5J is a plot of emulsifier-containing (TWEEN 20 non-ionic emulsifier) suspension storage time (measured in days along the "X" axis, indicated by reference numeral 801) vs. 10(log$_{10}$(emulsifier-containing suspension viscosity)-3) (measured along the "Y" axis, indicated by reference numeral 800) for the 16 data pairs for the suspension which is the subject of Figure 5G. Each of the graphs in Figures 5H, Figure 51 and Figure 5J indicated, respectively, by reference numeral 802 ,by reference numeral 804 and by reference numeral 805 includes each of the 16 data points of Figure 5G, one of which data point is indicated by reference numeral 803.

When the corresponding graphs of (a) Figure 5D together with Figure 5E, Figure 5F and Figure 20 (emulsifier-free suspension) vs. (b) Figure 5G together with Figure 5H, Figure 51 and Figure 5J (non-ionic emulsifier-containing suspension) are compared from the standpoint of rate of change of viscosity with respect to time (measured in centipoises/day at a shear rate of 1.0 seconds$^{-1}$ and at 25°C) the corresponding ratios "R" of

$$\left(\frac{\partial v}{\partial \theta}\right)_{w/oEM} : \left(\frac{\partial v}{\partial \theta}\right)_{withEM}$$

are as set forth in the following Table IV:

**Table IV**

| Figure Numbers and Graph Reference Numerals | Algorithm for $\left(\dfrac{\partial v}{\partial \theta}\right)_{w/oEM}$ | Algorithm for $\left(\dfrac{\partial v}{\partial \theta}\right)_{withEM}$ | Value of "R" |
|---|---|---|---|
| Figure 5E, reference numeral 602 and Figure 5H, reference numeral 802 | $\left(\dfrac{\partial v}{\partial \theta}\right) = 0.065v$ | $\left(\dfrac{\partial v}{\partial \theta}\right) = 0.0044$ | 14.77 |
| Figure 5F reference numeral 604 and Figure 5I, reference numeral 804 | $\left(\dfrac{\partial v}{\partial \theta}\right) = 0.125ve^{-0.028\theta}$ | $\left(\dfrac{\partial v}{\partial \theta}\right) = 0.0041ve^{0.0024\theta}$ | 25.7 at $\theta = 50$ |
| Figure 20, reference numeral 605 and Figure 5J reference numeral 805 | $\left(\dfrac{\partial v}{\partial \theta}\right) = 0.793\left(\dfrac{v}{\theta}\right)$ | $\left(\dfrac{\partial v}{\partial \theta}\right) = 0.21\left(\dfrac{v}{\theta}\right)$ | 3.78 |

wherein

$$\left(\frac{\partial v}{\partial \theta}\right)_{w/oEM}$$

represents the rate of change of viscosity with respect to time (measured in centipoises/day at a shear rate of 1.0 seconds$^{-1}$ and at 25°C) for the emulsifier-free suspension; wherein

$$\left(\frac{\partial v}{\partial \theta}\right)_{withEM}$$

represents the rate of change of viscosity with respect to time (measured in centipoises/day at a shear rate of 1.0 seconds$^{-1}$ and at 25°C) for the emulsifier-containing suspension; wherein $v$ represents viscosity (measured in centipoises at a shear rate of 1.0 seconds$^{-1}$ and at 25°C) and wherein $\theta$ represents time (measured in days).

The relative lack of increase in viscosity over an extended period of time and the substantially lower rate of change of viscosity with respect to time of the emulsifier-containing suspension of our invention when compared to suspensions of fragrance-containing microcapsules which have no emulsifier contained therein is indicative of an unobvious advantage of the suspension of our invention over the prior art.

Referring to Figure 6, fragrance component and/or benefit agent, e.g., malodour counteractant component formulation in storage tank 11 is passed through line 13 past control valve 14 into mixing vessel 17 equipped with agitator means 18, whereat is admixed with solvent, e.g., NEOBEE-M5 stored in tank 12 from which it is passed through line 15 past control valve 16 into mixing vessel 17 where an oil-phase, monophasic solvent/fragrance formulation and/or solvent/benefit agent, e.g., malodour counteractant formulation is prepared. Simultaneously, (i) substituted or unsubstituted acrylic acid polymer or co-polymer, e.g., an acrylic acid-acrylamide co-polymer, stored in tank 27 is passed through line 33 past control valve 34 into reactor 35 equipped with agitator means 36 and heating supply 37; and (ii) urea or melamine in storage vessel 19 is passed through line 21 past control valve 22 to reactor 25 where it is reacted with formaldehyde or formalin stored in tank 20 and passed through line 23 past control valve 24 into reactor 25, in order to form a urea-formaldehyde pre-condensate or melamine-formaldehyde pre-condensate which, in turn, is passed through line 28 past control valve 29 into reactor 35 (optionally, the pre-condensate may be partially etherified with a $C_1$-$C_6$ alkanol, in a reactor, not shown, located between valve 29 and reactor 35) together with water, originally stored in vessel 30, which is passed through line 31 past control valve 32, and aqueous acid pH adjustment composition which is originally stored in vessel 26, which is passed into reactor 35 through connecting line 80 equipped with control valve 83. An uncured substituted or un-substituted acrylic acid polymer or co-polymer, cross-linked with the urea-formaldehyde pre-condensate or melamine-formaldehyde pre-condensate is formed in reactor 35. A mixture of water and the resulting uncured substituted or un-substituted acrylic acid polymer or co-polymer, cross-linked with the urea-formaldehyde pre-condensate or melamine-formaldehyde pre-condensate is passed through line 40 past control valve 41 into homogenizer 42 where it is vigorously admixed with the solvent/fragrance formulation and/or solvent/benefit agent, e.g., malodour counteractant formulation which is passed from vessel 17 through line 38 past control valve 39. The homogenizer 42 is preferably of a type illustrated in Figures 11-A and 11-B of U.S. Patent 6,042,792 and described therein or Figures 7A, 7B, 7C and 7D. During the homogenization unit process, the un-cured substituted or un-substituted acrylic acid polymer or co-polymer, cross-linked with the urea-formaldehyde pre-condensate or melamine-formaldehyde pre-condensate (which is originally dispersed in the aqueous phase) is coacervated about each of the monophasic oil-phase droplets of the solvent/fragrance formulation and/or solvent/benefit agent, e.g., malodour counteractant formulation thereby forming filled microcapsules having uncured microcapsule shell walls, as shown in Figure 4B. The resulting uncured filled microcapsules in an aqueous slurry are passed from homogenizer 42 through line 43 past control valve 44 into curing vessel 48 which is equipped with heating means 49, and whereat the uncured filled microcapsules are cured at 50 - 85°C. Optionally, subsequent to the curing unit process, cationic coating polymer (e.g., U-RAMIN P-1500), stored in vessel 45 is passed through line 46 past control valve 47 into curing vessel 48 where the cationic polymer is coated onto the outer surface of each filled cured microcapsule. The cured, filled, optionally-coated microcapsule slurry is then passed through line 58 past control valve 59 into high shear mixer 60 (The high shear mixer is preferably of a type which is illustrated in Figures 8A and 8B having accessories of a type illustrated in Figures 8C, 8D, 8E, 8F, 8G and 8H). Non-confined fragrance or benefit agent, e.g., malodour counteractant or insect repellent composition, stored in vessel 50 is conveyed through line 52 past control valve 53 into mixing vessel 56 equipped with agitation means 57, whereat it is admixed with anionic, non-ionic and/or zwitterionic emulsifier stored in vessel 51, the emulsifier being conveyed to mixing vessel 56 through line 54 past control valve 55. The resulting non-confined fragrance and/or benefit agent, e.g., malodour counteractant or insect repellent-emulsifier mixture is then conveyed through line 61 past control valve 62 into the high shear mixer 60 where the suspension of our invention is crafted by mixing the non-confined fragrance and/or benefit agent, e.g., malodour counteractant or insect repellent-emulsifier mixture with the cured, filled, optionally-coated microcapsule slurry. The resulting suspension is then conveyed through line 64 past control valve 65 into storage vessel 66. The suspension, after storage is then utilized by means of passing the suspension through line 67 past control valve 68 into mixing vessel 70, equipped with agitating means, where it is admixed with a liquid consumable product such as model cationic fabric softener conditioner, stored at location 69 and conveyed through line 71 past valve 72 into mixing vessel 70. The resulting consumable material/stable suspension mixture is then, if desired, conveyed through line 74 into mixing vessel 75 equipped with agitation means 76 where it is further admixed with water which is stored in vessel 77 and conveyed through line 78 past control valve 79 into mixing vessel 75.

A preferred homogenizer, designated by reference numeral 42 in Figure 6 is a rotor/stator homogenizer as illustrated in Figures 7A, 7B, and 7C with the homogenizer assembly being illustrated in Figure 7D. Referring to Figure 7A the high speed rotation of the rotor blades 1106 within the precision machine mixing workhead exerts a powerful suction at location 1101 drawing (a) the liquid fragrance formulation and/or benefit agent, e.g., malodour counteractant or insect repellent and hydrophobic solvent entering the homogenization assembly from line 38 (shown in Figure 6) together with (b) the water and uncured acrylic acid polymer or co-polymer cross-linked with melamine-formaldehyde pre-condensate or urea-formaldehyde pre-condensate entering the homogenization assembly from line 40 (shown in Figure 6)(the entire mixture being indicated by reference numeral 1104a) into the rotor/stator assembly 1100. The rotation is effected at access 1102. The output from the assembly is at location 1103. The workhead is indicated by reference numeral 1105. The overall device is indicated by reference numeral 1100. Referring to Figure 7B, centrifugal force then drives materials 1104a towards a periphery of the workhead where the materials are subjected to an intense mixing action in the precision machined clearance the ends of the rotor blades 1106 and the inner wall of the stator. Referring to Figure 7C, stage "2" is followed by intense hydraulic shear as the materials 1104b are forced at high velocity out through the perforations in the stator 1106, then through the machine outlet and along the pipework 1103. At the same time, fresh materials are continually drawn into the workhead at 1101, maintaining the mixing and pumping cycle. Referring to Figure 7D, the homogenizing equipment assembly, mixer 1120, containing mixing shaft 1121 is a steam-heated feeder tank. The homogenizing equipment assembly is shown with a two stage pressure adjustment system when the first stage hand wheel is shown by reference numeral 1112. Pressure gauge 1122 is used to monitor the flow of microencapsulated fragrance and/or benefit agent slurry through a three-way bypass valve to cooling coils 1130 and recycle line 1114. Temperature gauge 1124 monitors the temperature of fluid flowing through line 1113 into the two-stage valve assembly which is attached to gear box 1123. The overall homogenizing equipment assembly is indicated by reference numeral 1110.

The high shear mixing apparatus of Figure 8A (prior to operation) and Figure 8B (during operation) is of the type provided by IKA-Werke GmbH & Co. KG Janke & Junkel Strasse, Staufen, Germany. The electric-powered dispersing instrument indicated by reference numeral 81 in Figure 8A and reference numeral 93 in Figure 8B (for example, the IKA T 50 basic ULTRA-TURRAX (Janke & Kunkel KG, IKA-Werke GmbH & Co.)) is secured to a rigid steel frame mounted on base 84 in Figure 8A and 95 in Figure 8B using boss head clamp 85 in Figure8A and 96 in Figure 8B and operates the high velocity rotating dispersing element 88 which is rotatably and securely mounted in element holder 82 in Figure 8A and 94 in Figure 8B which is vertically disposed within the confines of mixing vessel 87 in Figure 8A and 98 in Figure 8B. The mixing vessel is secured to the rigid steel frame using strap clamp 86 in Figure 8A and 97 in Figure 8B. Figure 8B illustrates the mixing of (a) the emulsifier/non-confined fragrance and/or benefit agent composition with (b) the cured fragrance and/or benefit agent-containing microcapsule/water mixture in order to create the stable suspension, 99, of our invention. Each of Figures 8C, 8D, 8E, 8F, 8G and 8H is an illustration of a utilizable dispersing element which can be used in place of dispersing element 88 in Figure 8A. Examples of IKA dispersing elements corresponding to those illustrated are set forth in Table V as follows:

Table V

| Figure | Reference Numeral | IKA Dispersing Element Number |
|---|---|---|
| 8C | 88c | S50N-G45G |
| 8D | 88d | S50N-G45M |
| 8E | 88e | S50N-G45P |
| 8F | 88f | S65KG-G65G |
| 88G | 88g | S65KG-G65M |
| 8H | 88h | S65KG-G65P |

For each of Figures 9, 10, 11, 12, 13, 14 and 15, the sensory intensities of aromas emitted from suspension-containing conditioner-treated towel sections (in accordance with the procedure of Example IV and referred to in the section entitled "BRIEF DESCRIPTION OF THE DRAWINGS) and 'controls' which are measurements of sensory intensities of aromas emitted from 'blanks' and from non-confined fragrance-treated towel sections (on a label magnitude scale of 0-99) is measured along the "Y" axis indicated by reference numeral 100; with the individual 'pre-rub' and 'post-rub' bar graphs being evenly distributed along the "X" axis, indicated by reference numeral 101. In all cases, the fragrance of Example B was employed.

The set of "2 week suspension storage" bar graphs of Figure 9 provides 'pre-rub' and 'post-rub' results using the procedure of Example IV. The particulars of the bar graphs with the mean scores are set forth in the following Table VI:

Table VI

| Emulsifier | Temperature | Presence of Non-Confined Fragrance of Example B | Presence of Microencapsulated Fragrance of Example B | 'Pre-Rub' Reference Numeral + Mean Score | 'Post-Rub' Reference Numeral + Mean Score |
|---|---|---|---|---|---|
| NONE | 25°C | yes | no | 102a/3.7 | 102b/1.9 |
| NONE | 25°C | yes | yes | 103a/4.2 | 103b/11.2 |
| Lecithin | 25°C | yes | yes | 104a/7.3 | 104b/13.4 |
| TWEEN 20 | 25°C | yes | yes | 105a/6.6 | 105b/11.8 |
| TWEEN 20 (Replicate) | 25°C | yes | yes | 106a/8.8 | 106b/11.8 |

Table VI   (continued)

| Emulsifier | Temperature | Presence of Non-Confined Fragrance of Example B | Presence of Microencapsulated Fragrance of Example B | 'Pre-Rub' Reference Numeral + Mean Score | 'Post-Rub' Reference Numeral + Mean Score |
|---|---|---|---|---|---|
| Sodium Lauryl Sulfate | 25°C | yes | yes | 107a/6.7 | 107b/15.3 |
| Lecithin | 25°C | yes | yes | 108a/6.7 | 108b/11.5 |
| TWEEN 20 | 37°C | yes | yes | 109a/7.2 | 109b/17.1 |
| TWEEN 20 (Replicate) | 37°C | yes | yes | 110a/4.3 | 10b/14.6 |
| Sodium Lauryl Sulfate | 37°C | yes | yes | 111a/10.1 | 111b/14.7 |
| None (blank) | 25°C | no | no | 145a/1.1 | 145b/0.9 |

The set of "4 week suspension storage" bar graphs of Figure 10 provides 'pre-rub' and 'post-rub' results using the procedure of Example IV. The particulars of the bar graphs with the mean scores are set forth in the following Table VII:

Table VII

| Emulsifier | Temperature | Presence of Non- Confined Fragrance of Example B | Presence of Microencapsulated Fragrance of Example B | 'Pre-Rub' Reference Numeral + Mean Score | 'Post-Rub' Reference Numeral + Mean Score |
|---|---|---|---|---|---|
| NONE (0 wk.) | 25°C | yes | no | 112a/5.2 | 112b/6.7 |
| NONE (0 wk.) | 25°C | yes | yes | 113a/9.2 | 113b/18.0 |
| Lecithin | 25°C | yes | yes | 114a/9.5 | 114b/18.0 |
| TWEEN 20 | 25°C | yes | yes | 115a/9.2 | 115b/17.8 |
| TWEEN 20 (Replicate) | 25°C | yes | yes | 116a/11.5 | 116b/19.4 |
| Sodium Lauryl Sulfate | 25°C | yes | yes | 117a/10.6 | 117b/18.3 |
| Lecithin | 25°C | yes | yes | 118a/9.1 | 118b/18.0 |
| TWEEN 20 | 37°C | yes | yes | 119a/9.1 | 119b/21.9 |
| TWEEN 20 (Replicate) | 37°C | yes | yes | 120a/12.1 | 120b/22.4 |
| Sodium Lauryl Sulfate | 37°C | yes | yes | 121a/7.8 | 121b/16.7 |
| None (blank) | 25°C | no | no | 122a/1.6 | 122b/0.8 |

The set of "8 week suspension storage" bar graphs of Figure 11 provides 'pre-rub' and 'post-rub' results using the procedure of Example IV, infra. The particulars of the bar graphs with the mean scores are set forth in the following Table VIII:

Table VIII

| Emulsifier | Temperature | Presence of Non-Confined Fragrance of Example B | Presence of Microencapsulated Fragrance of Example B | 'Pre-Rub' Reference Numeral + Mean Score | 'Post-Rub' Reference Numeral + Mean Score |
|---|---|---|---|---|---|
| NONE | 25°C | yes | no | 123a/10.0 | 123b/3.0 |
| NONE | 25°C | yes | yes | 124a/12.5 | 124b/22.4 |
| Lecithin | 25°C | yes | yes | 125a/12.6 | 125b/23.0 |
| Lecithin (Replicate) | 25°C | yes | yes | 126a/11.0 | 126b/14.2 |
| TWEEN 20 | 25°C | yes | yes | 127a/10.0 | 127b/15.0 |
| TWEEN 20 (Replicate) | 25°C | yes | yes | 128a/13.0 | 128b/20.1 |
| Lecithin | 37°C | yes | yes | 129a/11.1 | 129b/22.4 |
| Lecithin (Replicate) | 37°C | yes | yes | 130a/13.6 | 130b/20.8 |
| TWEEN 20 | 37°C | yes | yes | 131a/15.4 | 131b/17.7 |
| TWEEN 20 (Replicate) | 37°C | yes | yes | 132a/13.2 | 132b/19.4 |
| None (blank) | 25°C | no | no | 133a/1.8 | 133b/1.2 |

The set of "2 week fabric conditioner + suspension storage" bar graphs of Figure 12 provides 'pre-rub' and 'post-rub' results using the procedure of Example IV. An illustration of the indication of the standard deviation for the data is indicated by reference numeral 144c. The particulars of the bar graphs with the mean scores are set forth in the following Table IX:

Table IX

| Emulsifier | Temperature | Presence of Non- Confined Fragrance of Example B | Presence of Microencapsulated Fragrance of Example B | 'Pre-Rub' Reference Numeral + Mean Score | 'Post-Rub' Reference Numeral + Mean Score |
|---|---|---|---|---|---|
| NONE (2 wk.) | 25°C | yes | no | 134a/8.9 | 134b/4.4 |
| NONE (2 wk.) | 25°C | yes | yes | 135a/12.6 | 135b/21.3 |
| Lecithin | 25°C | yes | yes | 136a/8.9 | 136b/19.5 |
| Lecithin (Replicate) | 25°C | yes | yes | 137a/9.7 | 137b/19.4 |
| TWEEN 20 | 25°C | yes | yes | 138a/13.4 | 138b/18.1 |
| TWEEN 20 (Replicate) | 25°C | yes | yes | 139a/12.3 | 139b/15.1 |
| Lecithin | 37°C | yes | yes | 140a/7.6 | 140b/18.8 |
| Lecithin (Replicate) | 37°C | yes | yes | 141a/15.0 | 141b/16.6 |
| TWEEN 20 | 37°C | yes | yes | 142a/13.7 | 142b/19.7 |
| None (0 wk.) | 37°C | yes | yes | 143a/11.3 | 143b/21.9 |
| None (blank) | 25°C | no | no | 144a/0.9 | 144b/1.1 |

The details concerning the bar graphs in Figure 13 wherein the emulsifier used for crafting the suspension of our invention is sodium lauryl sulfate are set forth in the following Table X:

Table X

| Temperature | Storage Period of Suspension (weeks) | 'Pre-Rub' Reference Numeral +Mean Score | 'Post-Rub' Reference Numeral + Mean Score |
|---|---|---|---|
| 25°C | 2 | 107a/6.7 | 107b/15.3 |
| 37°C | 2 | 111a/10.1 | 111b/14.7 |
| 25°C | 4 | 117a/10.6 | 117b/18.3 |
| 37°C | 4 | 121a/7.8 | 121b/16.7 |

The details concerning the bar graphs in Figure 14 wherein the emulsifier used for crafting the suspension of our invention is TWEEN 20 are set forth in the following Table XI:

Table XI

| Temperature | Storage Period of Suspension (weeks) | 'Pre-Rub' Reference Numeral +Mean Score | 'Post-Rub' Reference Numeral + Mean Score |
|---|---|---|---|
| 25°C | 2 | 105a/6.6 | 105b/11.8 |
| 25°C | 2 | 106a/8.8 | 106b/11.8 |
| 37°C | 2 | 109a/7.2 | 109b/17.1 |
| 37°C | 2 | 110a/4.3 | 110b/14.6 |
| 25°C | 4 | 115a/9.2 | 115b/17.8 |
| 25°C | 4 | 116a/11.5 | 116b/19.4 |
| 37°C | 4 | 119a/9.1 | 119b/21.9 |
| 37°C | 4 | 120a/12.1 | 120b/22.4 |
| 25°C | 8 | 127a/10.0 | 127b/15.0 |
| 25°C | 8 | 128a/13.0 | 128b/20.1 |
| 37°C | 8 | 131a/15.4 | 131b/17.7 |
| 37°C | 8 | 132a/13.2 | 132b/19.4 |

The details concerning the bar graphs in Figure 15 wherein the emulsifier used for crafting the suspension of our invention is Lecithin are set forth in the following Table XII:

Table XII

| Temperature | Storage Period of Suspension (weeks) | 'Pre-Rub' Reference Numeral +Mean Score | 'Post-Rub' Reference Numeral + Mean Score |
|---|---|---|---|
| 25°C | 2 | 104a/7.3 | 104b/13.4 |
| 37°C | 2 | 108a/6.7 | 108b/11.5 |
| 25°C | 4 | 114a/9.5 | 114b/18.0 |
| 37°C | 4 | 118a/9.1 | 118b/18.0 |
| 25°C | 8 | 125a/12.6 | 125b/23.0 |
| 25°C | 8 | 126a/11.0 | 126b/14.2 |
| 37°C | 8 | 129a/ 11.1 | 129b/22.4 |
| 37°C | 8 | 130a/13.6 | 130b/20.8 |

Referring to Figures 16, 17, 18A, 18B and 19 wherein the data set forth in Figures 13, 14 and 15 is presented on each of said Figures 16 (data from Figure 13), 17 (data from Figure 14), 18A (data from Figure 15), 18B (data from Figure 15) and 19 (data from all of Figures 13, 14 and 15) as a plot of sensory intensity (on a scale of 0-99) on the "Y" axis (respectively indicated by reference numerals 160, 170,180, 180 and 190) vs. time (in weeks) on the "X" axis (respectively indicated by reference numerals 161, 171, 181, 181 and 191). The following Table XIII sets forth the specific emulsifier used for crafting the suspension of our invention, the specific figure relevant to that emulsifier, and the regression algorithm relevant to that emulsifier:

Table XIII

| Figure | Reference Numerals indicating graph and illustrative data point | Emulsifier | Regression Algorithm |
|---|---|---|---|
| 16 Data | Graph: 162 Point: 163 | sodium lauryl sulfate | $Y = 1.25X + 12.5$ |
| 17 Data | Graph:172 Point: 173 | TWEEN 20 | $Y = -0.64X^2 + 7.13X + 2.13$ |
| 18A | Graph: 182A Data Point: 183 | Lecithin | $Y = -0.375X^2 + 5.03X + 3.9$ |
| 18B | Graph: 182B Data Point: 183 | Lecithin | $Y = -17.13e^{-0.235X} + 24$ |
| 19 | Graph: 192 Data Point: 193 | sodium lauryl sulfate, TWEEN 20 and Lecithin (all data points are on the same plot) | $Y = -0.44X^2 + 5.28X + 4.98$ |

The following examples are not meant to define or otherwise limit the scope of the invention. Rather the scope of the invention is to be ascertained according to the claims that follow the examples. Unless noted to the contrary, all percentages are given on a weight percent on a dry basis.

## Example A

The following fragrance composition was prepared:

| Fragrance Component | C $\log_{10}P$ value | Molecular Weight | Parts by Weight |
|---|---|---|---|
| ethyl undecylenate | 4.888 | 212.34 | 3.0 |
| geranyl anthranilate | 4.216 | 273.38 | 7.5 |
| $\alpha$-irone | 3.820 | 206.33 | 6.3 |
| phenyl ethyl benzoate | 4.058 | 226.28 | 3.2 |
| d-limonene | 4.232 | 136.24 | 3.2 |
| cis-p-t-butylcyclohexyl acetate | 4.019 | 198.31 | 5.8 |
| amyl cinnamic aldehyde | 4.324 | 202.30 | 7.3 |
| hexyl cinnamic aldehyde | 5.473 | 216.33 | 12.6 |
| hexyl salicylate | 5.260 | 222.29 | 12.6 |

## Example B

The following fragrance composition was prepared:

| Fragrance Component | C $\log_{10}P$ value | Molecular Weight | Parts by Weight |
|---|---|---|---|
| $\beta$-phenyl ethanol | 1.183 | 122.17 | 2.6 |
| benzyl acetate | 1.960 | 150.17 | 1.5 |
| $\alpha$-irone | 3.820 | 206.33 | 6.3 |
| phenyl ethyl benzoate | 4.058 | 226.28 | 3.2 |

(continued)

| Fragrance Component | C log$_{10}$P value | Molecular Weight | Parts by Weight |
|---|---|---|---|
| d-limonene | 4.232 | 136.24 | 3.2 |
| cis-p-t-butylcyclohexyl acetate | 4.019 | 198.31 | 5.8 |
| amyl cinnamic aldehyde | 4.324 | 202.30 | 7.3 |
| hexyl cinnamic aldehyde | 5.473 | 216.33 | 12.6 |
| cis-jasmone | 2.712 | 164.25 | 14.3 |
| geraniol | 2.649 | 154.26 | 3.8 |
| hexyl salicylate | 5.260 | 222.29 | 12.6 |

## Example C

The following fragrance composition was prepared:

| Fragrance Component | Clog$_{10}$P value | Molecular Weight | Parts by Weight |
|---|---|---|---|
| 1-phenyl hexanol-5 | 3.299 | 178.28 | 3.4 |
| dihydromyrcenol | 3.03 | 156.27 | 2.8 |
| δ-undecalactone | 3.830 | 184.28 | 4.2 |
| amyl cinnamate | 3.771 | 218.30 | 4.2 |
| benzophenone | 3.120 | 182.22 | 2.3 |
| nerol | 2.649 | 154.25 | 3.3 |
| 2-methoxynaphthalene | 3.235 | 158.20 | 3.2 |
| ethyl undecylenate | 4.888 | 212.34 | 12.6 |
| geranyl anthranilate | 4.216 | 273.38 | 14.5 |
| α-irone | 3.820 | 206.33 | 10.7 |

## Example D

The following fragrance composition was prepared:

| Fragrance Component | Clog$_{10}$P value | Molecular Weight | Parts by Weight |
|---|---|---|---|
| α-santalol | 3.800 | 220.36 | 5.3 |
| iso-eugenol | 2.547 | 164.21 | 4.7 |
| amyl salicylate | 4.601 | 208.26 | 8.6 |
| benzyl salicylate | 4.383 | 228.25 | 8.8 |
| β-caryophyllene | 6.333 | 204.36 | 10.2 |
| cedrol | 4.530 | 222.37 | 10.2 |
| cedryl acetate | 5.436 | 264.41 | 10.2 |
| cedryl formate | 5.070 | 238.37 | 5.1 |
| cyclohexyl salicylate | 5.265 | 220.29 | 3.2 |
| γ-dodecalactone | 4.359 | 198.31 | 3.2 |

(continued)

| Fragrance Component | Clog$_{10}$P value | Molecular Weight | Parts by Weight |
|---|---|---|---|
| ethyl undecylenate | 4.888 | 212.34 | 3.2 |
| geranyl anthranilate | 4.216 | 273.38 | 4.6 |

## EXAMPLE I

## PREPARATION OF FRAGRANCE-CONTAINING MICROCAPSULES

50 parts by weight of the fragrance of Example A was admixed with 50 parts by weight of NEOBEE-M5 solvent thereby forming a 'fragrance/solvent composition'. In a homogenizer as illustrated in Figures 11-A and 11-B of U.S. Patent 6,042,792, and in Figures 7A, 7B, 7C and 7D, fragrance/solvent composition-containing microcapsules was prepared by interfacial polymerization of a microcapsule wall encapsulating fragrance/solvent composition droplets. To make the capsule slurry, a copolymer of acrylamide and acrylic acid was first dispersed in water together with a methylated melamine-formaldehyde pre-condensate. These two components were allowed to react under acidic conditions. The fragrance/solvent composition was then added into the solution and droplets of the desired size were achieved by high shear homogenization using the homogenization apparatus of Figures 7A, 7B, 7C and 7D. Curing of the polymeric layer around the fragrance/solvent composition droplets was achieved by increasing the temperature to 50-85°.C. The resulting capsule slurry contained 55% water, and 45% filled microcapsules (35% core consisting of 50% fragrance of Example A, and 50% NEOBEE M-5 and 10% microcapsule wall).

## EXAMPLE II

## PREPARATION OF CAPSULE PRODUCT (THE STABLE SUSPENSION OF THE INVENTION) WHICH CONTAINS BOTH ENCAPSULATED AND NON-CONFINED FRAGRANCE

An oil-in-water type emulsifier (TWEEN 20) was selected and added into neat fragrance oil prepared according to Example B at 2.5 weight % using an overhead mixer as illustrated in Figure 8A. The emulsifier-containing neat fragrance oil was homogenized with the slurry of capsules having shell walls composed of an acrylamide-acrylic acid co-polymer cross-linked with melamine-formaldehyde resin as described in Example I using a high shear mixer of the type illustrated in Figure 8A. Emulsifier-containing fragrance oil was added into capsule slurry at a weight ratio such that 1 part free fragrance to 1 part encapsulated fragrance was achieved in the final capsule product, the stable suspension of our invention.

## EXAMPLE III

## PREPARATION OF FABRIC CONDITIONER CONTAINING THE STABLE SUSPENSION OF THE INVENTION

A model cationic fabric conditioner containing a 5.0% cationic emulsifier composition (a 50:50 mixture of diethyl imidazoline quaternary ammonium chloride and distearyl dimethyl ammonium chloride) but not containing any fragrance was provided. The emulsifier-containing capsule product (that is, the stable suspension of our invention) of Example II containing 15 weight % encapsulated fragrance, 15 weight % non-confined fragrance, and 10% shell-wall material was added into the fabric conditioner base and mixed using an overhead agitator at 300 rpm until homogeneous. The capsule slurry and non-confined fragrance used to create the capsule product in Example II were added to the same fabric conditioner base separately. In each case, 0.2 weight % of non-confined fragrance and 0.2 weight % of encapsulated fragrance was used.

## EXAMPLE IV

## PERFORMANCE OF THE STABLE SUSPENSION OF THE INVENTION IN THE FABRIC CONDITIONER BASE

The fabric conditioner samples (110 grams per sample) referred to in Example III were introduced into a Sears, Roebuck and Co. KENMORE (Sears Brands LLC , Hoffman Estates, Illinois) washing machine during the rinse cycle thereof to condition 20 hand towels weighing a total of 2420 gm. Two rinse conditioner samples that contain 0.4 weight % ("control 1") and 1.2 weight % ("control 2") of non-confined fragrance were also used as controls. After rinsing, each of the hand towels, weighing 110 grams each, were line-dried for a period of 24 hours followed by sensory evaluation of 8 randomly-selected towels. The 8 randomly-selected dry towels were thus evaluated by a panel of ten people using the Label Magnitude Scale (LMS) from 0 to 99, wherein: 3 = "barely detectable"; 7 = "weak", 16 = "moderate", and 32 = "strong". Sensory scores were recorded before and after each of the eight randomly-selected towels each contained in a separate polyethylene bag were rubbed by hand. Each rubbing test took place employing 5 time intervals at 2 seconds per time interval for a total rubbing time of 10 seconds.

As will be observed from Table XIV, set forth below, the rinse conditioners containing the suspension of our invention evolved an aroma having a significantly greater post-rub intensity than either rinse conditioner containing solely non-confined fragrance; and no microencapsulated fragrance. Comparing the aroma intensity resulting from the utilization of the rinse conditioner containing the stable suspension of our invention with the aroma intensity resulting from the utilization of the rinse conditioner where the microencapsulated fragrance was added separately from the non-confined fragrance, no significant difference was noted. This demonstrates the suspension of our invention, that is, the combination of the microcapsule slurry plus non-confined fragrance mixture (including a non-ionic, anionic and/or zwitterionic emulsifier) performs advantageously and unexpectedly in fabric rinse conditioner compositions in a manner superior to that when non-confined fragrance is used alone in the fabric rinse conditioner.

Table XIV

| Nature of Additive to fabric conditioner base | Pre-rub sensory intensity rating | Post-rub sensory intensity rating |
|---|---|---|
| Non-confined fragrance (0.4 weight %): "Control 1" | 6.4 | 4.8 |
| Non-confined fragrance (1.2 weight %): "Control 2" | 9.5 | 9.9 |
| The suspension of our invention (microcapsule slurry + non-confined fragrance mixture (containing emulsifier) of Example II) | 7.1 | 14.2 |
| Microcapsule slurry + non-confined fragrance, each being added to the fabric conditioner base separately; and not as a unitary composition | 9.2 | 15.4 |

In the aforementioned examples, the fragrance composition of Example A may be replaced, yielding substantially the same results as those set forth in Example IV with any of the following compositions:

(i) The fragrance of Example B;
(ii) The fragrance of Example C;
(iii) The fragrance of Example D; and/or
(iv) A composition containing 95% by weight of the fragrance of Example A, Example B, Example C, Example D and 5% Aloe, Lanolin and/or Vitamin E.

All U.S. Patents and Patent Applications referenced herein are hereby incorporated by reference as if set forth in their entirety.

**Claims**

1.    A stable suspension having a viscosity of from about 100 to about 20,000 centipoises at a shear rate in the range of from about 0.5 to about 2 seconds$^{-1}$ and at about 25°C, the viscosity of which undergoes a minimal increase over an extended period of time, comprising (a) from about 10% by weight to about 90% by weight of a non-confined liquid-phase which is a substantially solid particle-free first fragrance composition and/or a substantially solid particle-free first benefit agent composition comprising from about 10% to about 90% by weight of a fragrance and/or benefit agent, from about 0.5% to about 100% of an emulsifier based on the weight of the non-confined fragrance and from about 10% to about 90% water, in the form of a stable oil-in-water emulsion and (b) stably suspended in said non-confined liquid-phase from about 10% to about 90% by weight of a plurality of microcapsules each of which (i) has an outside diameter in the range of from about 0.01 to about 1000 microns; (ii) has a wall thickness in the range of from about 0.001 to about 100 microns; (iii) has a wall composed of a polymer; and (iv) has a liquid phase core comprising a - second fragrance composition and/or second benefit agent composition with the composition of each of the cores of each of said microcapsules being (A) the same and/or different from one another and (B) the same or different from the first fragrance composition and/or first benefit agent composition wherein the weight % of second fragrance composition and/or substantially solid particle-free second benefit agent composition initially contained in each of the microcapsules is from about 5% to 90% by weight of the microcapsules.

2.  The stable suspension of claim 1 having a viscosity of from about 1000 centipoises to about 15,000 centipoises at a shear rate in the range of from about 0.5 to about 2 seconds$^{-1}$ and at about 25°C.

3.  The stable suspension of claim 1 having a viscosity of from about 2000 centipoises to about 12,000 centipoises at a shear rate in the range of from about 0.5 to about 2 seconds$^{-1}$ and at about 25°C.

4.  The stable suspension of claim 1 wherein the emulsifier contained in the non-confined liquid phase is at least one emulsifier selected from the group consisting of non-ionic emulsifers, anionic emulsifiers and zwitterionic emulsifiers, each of which has an HLB value of from about 6 to about 40 with the provisos that:

    (a) when using a non-ionic emulsifier, the HLB value is in the range of from about 6 to about 20;
    (b) when using an anionic emulsifier, the HLB value is in the range of from about 10 to about 40; and
    (c) when using a zwitterionic emulsifier, the HLB value is in the range of from about 6 to about 12.

5.  The stable suspension of claim 4 wherein the emulsifier is a non-ionic emulsifier having a HLB value in the range of from about 6 to about 20.

6.  The stable suspension of claim 5 wherein the non-ionic emulsifier is polyoxyethylene (20) sorbitan monolaurate.

7.  The stable suspension of claim 4 wherein the emulsifier is a zwitterionic emulsifier having a HLB value in the range of from about 6 to about 12.

8.  The stable suspension of claim 7 wherein the zwitterionic emulsifier is a phosphatidylcholine.

9.  The stable suspension of claim 4 wherein the emulsifier is an anionic emulsifier having a HLB value in the range of from about 10 to about 40.

10. The stable suspension of claim 9 wherein the anionic emulsifier is the sodium salt of n-dodecyl sulfate.

11. The stable suspension of claim 1 wherein said non-confined liquid phase consists essentially of a first fragrance composition, water and an emulsifier having a HLB value of from about 6 to about 40 with the provisos:

    (a) when using a non-ionic emulsifier, the HLB value is in the range of from about 6 to about 20;
    (b) when using an anionic emulsifier, the HLB value is in the range of from about 10 to about 40; and
    (c) when using a zwitterionic emulsifier, the HLB value is in the range of from about 6 to about 12;

    and the core of each of said plurality of microcapsules consists essentially of a second fragrance composition and/or a second malodour counteractant composition in admixture with a solvent.

12. The stable suspension of claim 1 wherein the wall of each of said plurality of microcapsules is composed of a substituted or un-substituted acrylic acid polymer or co-polymer cross-linked with a melamine-formaldehyde pre-condensate or a urea-formaldehyde pre-condensate.

13. The stable suspension of claim 11 wherein the solvent is selected from the group consisting of a mono-, di- or tri-$C_4$-$C_{26}$ saturated or unsaturated fatty acid glyceride, diethyl phthalate, dibutyl phthalate, diisodecyl adipate, a liquid polydimethyl siloxane, a liquid polydimethylcyclosiloxane, the methyl ester of soya fatty acid, a mixture of soya fatty acid methyl ester and isopropyl myristate with the weight ratio of soya fatty acid:isopropyl myristate being from 2:1 to 20:1 and a mineral oil compatible with each component of said second fragrance composition and/or said second malodour counteractant composition.

14. The stable suspension of claim 11 wherein each of the microcapsules has an average diameter in the range of from about 0.05 microns to about 100 microns and an average wall thickness in the range of from about 0.005 microns to about 10 microns.

15. The stable suspension of claim 11 wherein each of the microcapsules has an average diameter in the range of from about 2.0 microns to about 20 microns and an average wall thickness in the range of from about 0.2 microns to about 2.0 microns.

16. The stable suspension of claim 11 wherein all of the components of the solvent components have a C $\log_{10}$P greater than about 8.

17. The stable suspension of claim 11 wherein all of the components of the solvent components have a C $\log_{10}$P greater than about 10.

18. The stable suspension of claim 1 wherein each of the microcapsules contains said second fragrance composition in admixture with a solvent composition and is prepared according to a process comprising the steps of:

(i) providing a product base containing non-confined first fragrance composition and emulsifier material;
(ii) providing a permeable capsule wherein the permeable capsule contains second fragrance composition and/ or a compatible high C $\log_{10}$ P solvent having a C $\log_{10}$ P value of greater than about 3.3; and
(iii) allowing the non-encapsulated second fragrance composition and/ or solvent composition to come to equilibrium thereby transporting a portion of the non-confined first fragrance composition through the permeable shell wall into the interior of the capsule and retaining the fragrance contents in the permeable capsule.

19. The stable suspension of claim 1 wherein each of the microcapsules is a permeable microcapsule containing at least 20 weight percent sacrificial solvent capable of migrating outside of the capsule over a period of time in the range of from about 50 hours to about 200 hours.

20. The stable suspension of claim 19 wherein the sacrificial solvent contained in the microcapsules is selected from the group consisting of benzyl acetate and n-octanol.

21. The stable suspension of claim 1 wherein each of the microcapsules is produced according to the process comprising the steps of:

(i) providing a sacrificial solvent having a C $\log_{10}$ P value of from about 1 to about 3;
(ii) encapsulating the sacrificial solvent with a permeable encapsulate material;
(iii) providing the encapsulated sacrificial solvent in a liquid environment containing high C $\log_{10}$ P fragrance components with C $\log_{10}$ P of greater than about 3.3; and
(iv) allowing the capsules containing the sacrificial solvent to come to equilibrium

with the environment containing the high C $\log_{10}$ P fragrance components; whereby at least 20 weight percent of the sacrificial solvent migrates from the capsule into the environment.

22. The stable suspension of claim 1 wherein the non-confined liquid phase also contains a substance selected from the group consisting of at least one deposition aid, at least one additional surfactant, at least one humectant, at least one viscosity control agent and at least one solvent.

23. The stable suspension of claim 1 further comprising a substance selected from the group consisting of from about 0.1% to about 50% of at least one deposition aid, from about 0.1 % to about 50% of at least one additional surfactant, from about 0.1% to about 50% of at least one humectant, from about 0.1 % to about 20% of at least one viscosity control agent and from about 0.1 % to about 50% of at least one solvent.

24. The stable suspension of claim 1 wherein at least a finite portion of said microcapsules is coated with a cationic polymer.

25. The stable suspension of claim 1 wherein the liquid phase core of at least a finite portion of the microcapsules comprises a hydrophobic benefit agent selected from the group consisting of lanolin, aloe and Vitamin E.

26. A process for imparting a benefit or an aroma to a consumable material selected from the group consisting of liquid anionic, cationic, non-ionic or zwitterionic detergents, shampoos, bodywashes, soaps, hair conditioners, skin lotions, anti-perspirants, deodorants and fabric softener and/or conditioner compositions comprising the step of adding to said consumable material an aroma or benefiting amount of the stable suspension defined according to claim 1.

27. A process for preparing the stable suspension of claim 1 comprising the steps of (a) providing an aqueous slurry of a plurality of microcapsules having a polymeric wall and a core comprising a first fragrance composition and/or at least one first benefit agent; (b) admixing an emulsifier having a HLB value of from about 6 to about 40 with the provisos that:

(a) when using a non-ionic emulsifier the, HLB value is in the range of from about 6 to about 20;
(b) when using an anionic emulsifier, the HLB value is in the range of from about 10 to about 40; and
(c) when using a zwitterionic emulsifier, the HLB value is in the range of from about 6 to about 12;

with a second hydrophobic fragrance composition and/or a second hydrophobic benefit agent thereby forming a surfactant-second fragrance and/or second benefit agent mixture; and (c) admixing the aqueous slurry with the surfactant-second fragrance and/or second benefit agent mixture.

28. The process of claim 27 wherein the wall of each of the microcapsules is composed of a substituted or un-substituted acrylamide-acrylic acid co-polymer cross-linked with a melamine-formaldehyde and/or a urea-formaldehyde pre-condensate.

29. The stable suspension of claim 1 wherein the emulsifier is present at a level in the range of from about 1% to about 10% by weight based on the weight of non-confined fragrance.

30. The stable suspension of claim 29 wherein the emulsifier is present at a level of about 2.5% by weight based on the weight of non-confined fragrance.

31. The stable suspension of claim 1 wherein the relationship of the viscosity of the suspension with respect to time of storage of said suspension immediately subsequent to the production of said suspension is according to the set of algorithms selected from the group consisting of:

(i) $\log_e v = \alpha\theta + \beta$ and $\frac{\partial v}{\partial \theta} = \alpha v$;

(ii) $\log_e v = \gamma e^{\delta\theta} + \varepsilon$ and $\frac{\partial v}{\partial \theta} = v\delta\gamma e^{\delta\theta}$; and

(iii)

$$\log_e v = \kappa \log_e \theta + \lambda \text{ and } \frac{\partial v}{\partial \theta} = \kappa\left(\frac{v}{\theta}\right)$$

wherein:

$0.003 \le \alpha \le 0.006$;
$7 \le \beta \le 10$;
$1 \le \gamma \le 3$;
$0.002 \le \delta \le 0.003$;
$6 \le \varepsilon \le 8$;
$0.15 \le \kappa \le 0.25$; and
$7 \le \lambda \le 9$

and wherein $v$ represents the viscosity of said suspension in units of centipoises and $\theta$ represents the time of storage of said suspension immediately subsequent to production of said suspension, in terms of days.

**32.** Apparatus for carrying out the process of claim 28 comprising:

i. slurry preparation means for preparing a slurry of microencapsulated fragrance and/or benefit agent in water comprising (a) homogenization means, (b) fragrance and/or benefit agent-hydrophobic solvent first mixing means which is associated with and upstream from said homogenization means and (c) polymer-cross-linking agent reaction means which is associated with and upstream from said homogenization means, and (d) microcapsule wall curing means for forming cured microencapsulated fragrance and/or benefit agent downstream from and associated with said homogenization means;

ii. high shear second mixing means downstream from and associated with said curing means in which said stable suspension is formed;

iii. means for introduction of said cured microencapsulated fragrance and/or benefit agent from said curing means into said high shear second mixing means;

iv. third mixing means apart from said slurry preparation means for mixing emulsifier and non-confined fragrance and/or benefit agent, whereby a second mixture is formed;

v. means for second mixture introduction into said high shear second mixing means; and

vi. optional storage means for storing said stable suspension formed in said high shear second mixing means, said optional storage means being located downstream from and associated with said high shear second mixing means.

**33.** The stable suspension of claim 14 wherein each of the oil phase component droplets of the emulsion containing non-confined fragrance and/or benefit agent has a diameter in the range of from about 0.01 microns to about 1.0 microns.

**34.** The stable suspension of claim 33 wherein each of the oil phase component droplets of the emulsion containing non-confined fragrance and/or benefit agent has a diameter of from about 0.05 microns to about 0.8 microns.

**35.** The stable suspension of claim 34 wherein each of the oil phase component droplets of the emulsion containing non-confined fragrance and/or benefit agent has a diameter of from about 0.1 microns to about 0.5 microns.

**36.** The stable suspension of claim 1 wherein the emulsifier contained in the non-confiend liquid phase is a polymeric emulsifier used either alone or in combination with the emulsifiers selected from the group consisting of non-ionic emulsifers, anionic emulsifiers and zwitterionic emulsifiers, each of which has an HLB value of from about 6 to about 40 with the provisos that:

(a) when using a non-ionic emulsifier, the HLB value is in the range of from about 6 to about 20;

(b) when using an anionic emulsifier, the HLB value is in the range of from about 10 to about 40; and

(c) when using a zwitterionic emulsifier, the HLB value is in the range of from about 6 to about 12.

**37.** The stable suspension of claim 29 wherein the emulsifier is a polymeric emulsifier selected from the group consisting of modified starch, gum arabic and cross linked copolymers of acrylic acid and a hydrophobic comonomer.

4

FIG. 1

4

6

8

FIG. 2

4

6

8

9

FIG. 3

6

X

Y

8

$(X-Y)_n$

FIG. 4A

6

X + Y

$(X-Y)_n$

$(X-Y)_n$

8

FIG. 4B

FIG. 5A

FIG. 5B

FIG. 5C

FIG. 5D

EP 1 589 092 A1

10(LOG$_{10}$[VISCOSITY]-3)

FIG. 5E

10(LOG$_{10}$[VISCOSITY]-3)

FIG. 5F

FIG. 5G

FIG. 5H

FIG. 5I

FIG. 5J

FIG. 6

FIG. 7A

FIG. 7B

FIG. 7C

## FIG. 7D

FIG. 8A

FIG. 8B

FIG. 8C

FIG. 8D

FIG. 8E

FIG. 8F

FIG. 8G

FIG. 8H

FIG. 9

FIG. 10

FIG. 11

FIG. 12

EP 1 589 092 A1

FIG. 13

FIG. 14

FIG. 15

EP 1 589 092 A1

FIG. 16

FIG. 17

FIG. 18A

FIG. 18B

SENSORY
INTENSITY

"Y" AXIS

24
20
16
12
8
4

190

192

193

1  2  3  4  5  6  7  8  9

TIME (WEEKS)

191  "X" AXIS

## FIG. 19

$10(LOG_{10}[VISCOSITY]-3)$

"Y"
AXIS

16
12
8
4

600

603

605

10   20   30   40

TIME (DAYS)

601   "X" AXIS

## FIG. 20

**European Patent Office**

## EUROPEAN SEARCH REPORT

Application Number

EP 05 25 2298

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 231 873 B1 (NODA AKIRA ET AL) 15 May 2001 (2001-05-15) * column 15, lines 6,7; examples * * column 19, lines 45-63 * * column 12, lines 49-52 * ----- | 1-37 | C11D3/00 B01J13/00 A61K7/50 C11D3/50 A61K7/16 A61K7/46 |
| X | US 2002/022038 A1 (BIATRY BRUNO ET AL) 21 February 2002 (2002-02-21) * claims; examples 3,7 * ----- | 1-37 | |
| X | US 6 042 792 A (SHEFER ET AL) 28 March 2000 (2000-03-28) * column 32, line 65 - column 35, line 17; claims; figures 12A-12D; examples * * column 4, lines 54-59 * ----- | 1-37 | |
| D,X | US 4 464 271 A (MUNTEANU ET AL) 7 August 1984 (1984-08-07) | 32 | |
| A | * figure 3; examples * | 1-31, 33-37 | |
| A | ----- US 2003/045447 A1 (HEIBEL MARIJA ET AL) 6 March 2003 (2003-03-06) * tables 1-5 * ----- | 1-37 | TECHNICAL FIELDS SEARCHED (Int.Cl.7) C11D B01J A61K |
| A | US 5 112 688 A (MICHAEL ET AL) 12 May 1992 (1992-05-12) * examples * ----- | 1-37 | |

The present search report has been drawn up for all claims

| Place of search | Date of completion of the search | Examiner |
|---|---|---|
| Munich | 20 July 2005 | Miller, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 05 25 2298

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2005

| Patent document cited in search report | | Publication date | Patent family member(s) | | Publication date |
|---|---|---|---|---|---|
| US 6231873 | B1 | 15-05-2001 | JP | 62161712 A | 17-07-1987 |
| | | | JP | 1125313 A | 17-05-1989 |
| | | | JP | 2097182 C | 02-10-1996 |
| | | | JP | 7121850 B | 25-12-1995 |
| | | | JP | 1238513 A | 22-09-1989 |
| | | | JP | 2639816 B2 | 13-08-1997 |
| | | | JP | 1265007 A | 23-10-1989 |
| | | | JP | 1266846 A | 24-10-1989 |
| | | | JP | 2700069 B2 | 19-01-1998 |
| | | | JP | 1268620 A | 26-10-1989 |
| | | | JP | 1268621 A | 26-10-1989 |
| | | | JP | 1268622 A | 26-10-1989 |
| | | | DE | 3882906 D1 | 09-09-1993 |
| | | | DE | 3882906 T2 | 23-12-1993 |
| | | | EP | 0316054 A1 | 17-05-1989 |
| | | | US | 5089269 A | 18-02-1992 |
| US 2002022038 | A1 | 21-02-2002 | CA | 2346320 A1 | 05-11-2001 |
| | | | CN | 1323579 A ,C | 28-11-2001 |
| | | | EP | 1151741 A1 | 07-11-2001 |
| | | | JP | 2001354551 A | 25-12-2001 |
| US 6042792 | A | 28-03-2000 | CA | 2246639 A1 | 18-03-1999 |
| | | | DE | 908174 T1 | 17-02-2000 |
| | | | EP | 0908174 A2 | 14-04-1999 |
| | | | ES | 2133260 T1 | 16-09-1999 |
| | | | ID | 22071 A | 02-09-1999 |
| | | | JP | 11171754 A | 29-06-1999 |
| | | | SG | 75872 A1 | 24-10-2000 |
| | | | US | 6156826 A | 05-12-2000 |
| | | | US | 6645479 B1 | 11-11-2003 |
| US 4464271 | A | 07-08-1984 | US | 4428869 A | 31-01-1984 |
| | | | US | 4446032 A | 01-05-1984 |
| US 2003045447 | A1 | 06-03-2003 | CA | 2451225 A1 | 09-01-2003 |
| | | | GB | 2394726 A ,B | 05-05-2004 |
| | | | WO | 03002699 A1 | 09-01-2003 |
| US 5112688 | A | 12-05-1992 | AU | 638972 B2 | 15-07-1993 |
| | | | AU | 5016390 A | 30-08-1990 |
| | | | CA | 2009047 A1 | 27-08-1990 |
| | | | DE | 69027232 D1 | 11-07-1996 |
| | | | DE | 69027232 T2 | 23-01-1997 |
| | | | EG | 18744 A | 28-02-1994 |
| | | | EP | 0385535 A1 | 05-09-1990 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 05 25 2298

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2005

| Patent document cited in search report | Publication date | Patent family member(s) | Publication date |
|---|---|---|---|
| US 5112688 A | | ES 2087884 T3 | 01-08-1996 |
| | | JP 3202142 A | 03-09-1991 |
| | | MA 21755 A1 | 01-10-1990 |
| | | NZ 232680 A | 26-08-1992 |
| | | PT 93226 A | 31-08-1990 |
| | | TR 27499 A | 07-06-1995 |

EPO FORM P0459

For more details about this annex : see Official Journal of the European Patent Office, No. 12/82